# EUROPEAN PATENT SPECIFICATION

(11) **EP 2 843 047 B1**
(45) Date of publication and mention of the grant of the patent: **30.10.2019**
(21) Application number: 13755881.3
(22) Date of filing: 27.02.2013
(51) Int. Cl.: C12N 15/09, C12Q 1/68

(54) **NUCLEIC ACID DETECTION METHOD**
NUCLEINSÄUREDETEKTIONSVERFAHREN
PROCÉDÉ DE DÉTECTION D'ACIDES NUCLÉIQUES

(30) Priority: 27.02.2012 JP 2012040051
(43) Date of publication of application: 04.03.2015
(73) Proprietor: Toray Industries, Inc., Tokyo 103-8666 (JP)
(72) Inventor: UEDA, Yoji, Kamakura-shi Kanagawa 248-8555 (JP); NAKAMURA, Fumio, Kamakura-shi Kanagawa 248-8555 (JP)
(74) Representative: Kador & Partner PartG mbB
(86) International application number: PCT/JP2013/055085
(87) International publication number: WO 2013/129457

(56) References cited:
- WO-A1-95/09248
- WO-A1-96/31622
- WO-A1-99/28494
- WO-A1-99/67628
- WO-A1-2007/034897
- WO-A2-2004/001067
- WO-A2-2005/047474
- JP-A- H02 502 250
- JP-A- H05 501 957
- JP-A- S58 501 703
- JP-A- S62 229 068
- JP-A- 2004 502 464
- JP-A- 2007 535 944
- US-A1- 2011 105 348
- US-B1- 6 268 147
- OAEW S. ET AL.: 'Sensitivity enhancement in DNA hybridization assay using gold nanoparticle-labeled two reporting probes' BIOSENS. BIOELECTRON. vol. 25, no. 2, 2009, pages 435 - 441, XP026600446

### SEQUENCE LISTING

<110> TORAY INDUSTRIES, INC.
<120> Method of detecting nucleic acids
<130> PF477-PCT
<160> 47
<170> PatentIn version 3.5
<210> 1
   <211> 30
   <212> DNA
   <213> Human papillomavirus type 6
<400> 1
   atccgtaact acatcttcca catacaccaa 30
<210> 2
   <211> 30
   <212> DNA
   <213> Human papillomavirus type 11
<400> 2
   atctgtgtct aaatctgcta catacactaa 30
<210> 3
   <211> 30
   <212> DNA
   <213> Human papillomavirus type 16
<400> 3
   gtcattatgt gctgccatat ctacttcaga 30
<210> 4
   <211> 30
   <212> DNA
   <213> Human papillomavirus type 18
<400> 4
   tgcttctaca cagtctcctg tacctgggca 30
<210> 5
   <211> 30
   <212> DNA
   <213> Human papillomavirus type 31
<400> 5
   tgtttgtgct gcaattgcaa acagtgatac 30
<210> 6
   <211> 30
   <212> DNA
   <213> Human papillomavirus type 33
<400> 6
   tttatgcaca caagtaacta gtgacagtac 30
<210> 7
   <211> 30
   <212> DNA
   <213> Human papillomavirus type 34
<400> 7
   tacacaatcc acaagtacaa atgcaccata 30
<210> 8
   <211> 30
   <212> DNA
   <213> Human papillomavirus type 35
<400> 8
   gtctgtgtgt tctgctgtgt cttctagtga 30
<210> 9
   <211> 30
   <212> DNA
   <213> Human papillomavirus type 39
<400> 9
   tctacctcta tagagtcttc cataccttct 30
<210> 10
   <211> 30
   <212> DNA
   <213> Human papillomavirus type 40
<400> 10
   gctgccacac agtcccccac accaacccca 30
<210> 11
   <211> 30
   <212> DNA
   <213> Human papillomavirus type 42
<400> 11
   ctgcaacatc tggtgataca tatacagctg 30
<210> 12
   <211> 30
   <212> DNA
   <213> Human papillomavirus type 43
<400> 12
   tctactgacc ctactgtgcc cagtacatat 30
<210> 13
   <211> 30
   <212> DNA
   <213> Human papillomavirus type 44
<400> 13
   gccactacac agtcccctcc gtctacatat 30
<210> 14
   <211> 30
   <212> DNA
   <213> Human papillomavirus type 45
<400> 14
   acacaaaatc ctgtgccaag tacatatgac 30
<210> 15
   <211> 30
   <212> DNA
   <213> Human papillomavirus type 51
<400> 15
   agcactgcca ctgctgcggt ttccccaaca 30
<210> 16
   <211> 30
   <212> DNA
   <213> Human papillomavirus type 52
<400> 16
   tgctgaggtt aaaaaggaaa gcacatataa 30
<210> 17
   <211> 30
   <212> DNA
   <213> Human papillomavirus type 54
<400> 17
   tacagcatcc acgcaggata gctttaataa 30
<210> 18
   <211> 30
   <212> DNA
   <213> Human papillomavirus type 56
<400> 18
   gtactgctac agaacagtta agtaaatatg 30
<210> 19
   <211> 30
   <212> DNA
   <213> Human papillomavirus type 58
<400> 19
   attatgcact gaagtaacta aggaaggtac 30
<210> 20
   <211> 20
   <212> DNA
   <213> human papillomavirus
<220>
   <221> misc_feature
   <222> (1)..(1)
   <223> biotinylated base
<220>
   <221> misc_feature
   <222> (20)..(20)
   <223> biotinylated base
<400> 20
   gcmcagggwc ataayaatgg 20
<210> 21
   <211> 25
   <212> DNA
   <213> human papillomavirus
<220>
   <221> misc_feature
   <222> (1)..(1)
   <223> biotinylated base
<220>
   <221> misc_feature
   <222> (25)..(25)
   <223> biotinylated base
<400> 21
   gaaaaataaa ctgtaaatca tattc 25
<210> 22
   <211> 23
   <212> DNA
   <213> human papillomavirus
<220>
   <221> misc_feature
   <222> (1)..(1)
   <223> biotinylated base
<220>
   <221> misc_feature
   <222> (23)..(23)
   <223> biotinylated base
<400> 22
   tttgttactg tggtagatac tac 23
<210> 23
   <211> 20
   <212> DNA
   <213> human papillomavirus
<220>
   <221> misc_feature
   <222> (1)..(1)
   <223> biotinylated base
<220>
   <221> misc_feature
   <222> (20)..(20)
   <223> biotinylated base
<400> 23
   gatcagtwtc cyytdggacg 20
<210> 24
   <211> 80
   <212> DNA
   <213> human papillomavirus
<220>
   <221> misc_feature
   <222> (10)..(10)
   <223> n is biotinylated base
<220>
   <221> misc_feature
   <222> (30)..(30)
   <223> n is biotinylated base
<220>
   <221> misc_feature
   <222> (51)..(51)
   <223> n is biotinylated base
<220>
   <221> misc_feature
   <222> (71)..(71)
   <223> n is biotinylated base
<400> 24
   gcacagggcn acaataatgg catttgttgn ggtaaccaac tatttgttac ngttgttgat     60
   actacacgca ntacaaatat                                                 80
<210> 25
   <211> 86
   <212> DNA
   <213> human papillomavirus
<220>
   <221> misc_feature
   <222> (10)..(10)
   <223> n is biotinylated base
<220>
   <221> misc_feature
   <222> (30)..(30)
   <223> n is biotinylated base
<220>
   <221> misc_feature
   <222> (57)..(57)
   <223> n is biotinylated base
<220>
   <221> misc_feature
   <222> (77)..(77)
   <223> n is biotinylated base
<400> 25
   aactacatan aaaaatacta actttaaggn gtacctacga catggggagg aatatgnttt     60
   acagtttatt tttcaantgt gcaaaa                                          86
<210> 26
   <211> 86
   <212> DNA
   <213> human papillomavirus
<220>
   <221> misc_feature
   <222> (10)..(10)
   <223> n is biotinylated base
<220>
   <221> misc_feature
   <222> (30)..(30)
   <223> n is biotinylated base
<220>
   <221> misc_feature
   <222> (50)..(50)
   <223> n is biotinylated base
<220>
   <221> misc_feature
   <222> (70)..(70)
   <223> n is biotinylated base
<400> 26
   taaccttaan tgcagacgtt atgacatacn tacattctat gaattccacn attttggagg 60
   actggaattn tggtctacaa cctccc 86
<210> 27
   <211> 86
   <212> DNA
   <213> human papillomavirus
<220>
   <221> misc_feature
   <222> (10)..(10)
   <223> n is biotinylated base
<220>
   <221> misc_feature
   <222> (30)..(30)
   <223> n is biotinylated base
<220>
   <221> misc_feature
   <222> (57)..(57)
   <223> n is biotinylated base
<220>
   <221> misc_feature
   <222> (77)..(77)
   <223> n is biotinylated base
<400> 27
   ccaggaggcn cactagaaga tacttatagn tttgtaacat cccaggcaat tgcttgncaa 60
   aaacatacac ctccagnacc taaaga 86
<210> 28
   <211> 84
   <212> DNA
   <213> human papillomavirus
<220>
   <221> misc_feature
   <222> (10)..(10)
   <223> n is biotinylated base
<220>
   <221> misc_feature
   <222> (30)..(30)
   <223> n is biotinylated base
<220>
   <221> misc_feature
   <222> (55)..(55)
   <223> n is biotinylated base
<220>
   <221> misc_feature
   <222> (75)..(75)
   <223> n is biotinylated base
<400> 28
   agatcccctn aaaaaataca ctttttgggn agtaaattta aaggaaaagt tttcngcaga 60
   cctagatcag tttcntttag gacg 84
<210> 29
   <211> 21
   <212> DNA
   <213> Homo sapiens
<400> 29
   agttggagct ggtggcgtag g 21
<210> 30
   <211> 21
   <212> DNA
   <213> Homo sapiens
<400> 30
   agttggagct agtggcgtag g 21
<210> 31
   <211> 21
   <212> DNA
   <213> Homo sapiens
<400> 31
   agttggagct cgtggcgtag g 21
<210> 32
   <211> 21
   <212> DNA
   <213> Homo sapiens
<400> 32
   agttggagct tgtggcgtag g 21
<210> 33
   <211> 21
   <212> DNA
   <213> Homo sapiens
<400> 33
   caactcatca cgcagctcat g 21
<210> 34
   <211> 21
   <212> DNA
   <213> Homo sapiens
<400> 34
   caactcatca tgcagctcat g 21
<210> 35
   <211> 21
   <212> DNA
   <213> Homo sapiens
<400> 35
   caactcatca agcagctcat g 21
<210> 36
   <211> 21
   <212> DNA
   <213> Homo sapiens
<400> 36
   caactcatca ggcagctcat g 21
<210> 37
   <211> 40
   <212> DNA
   <213> Homo sapiens
<220>
   <221> misc_feature
   <222> (1)..(1)
   <223> biotinylated base
<220>
   <221> misc_feature
   <222> (40)..(40)
   <223> biotinylated base
<400> 37
   gatcatattc gtccacaaaa tgattctgaa ttagctgtat 40
<210> 38
   <211> 40
   <212> DNA
   <213> Homo sapiens
<220>
   <221> misc_feature
   <222> (1)..(1)
   <223> biotinylated base
<220>
   <221> misc_feature
   <222> (40)..(40)
   <223> biotinylated base
<400> 38
   ccaagagaca ggtttctcca tcaattacta cttgcttcct 40
<210> 39
   <211> 40
   <212> DNA
   <213> Homo sapiens
<220>
   <221> misc_feature
   <222> (1)..(1)
   <223> biotinylated base
<220>
   <221> misc_feature
   <222> (40)..(40)
   <223> biotinylated base
<400> 39
   tcctcatgta ctggtccctc attgcactgt actcctcttg 40
<210> 40
   <211> 40
   <212> DNA
   <213> Homo sapiens
<220>
   <221> misc_feature
   <222> (1)..(1)
   <223> biotinylated base
<220>
   <221> misc_feature
   <222> (40)..(40)
   <223> biotinylated base
<400> 40
   aattttaact ttctcacctt ctgggatcca gagtccctta 40
<210> 41
   <211> 40
   <212> DNA
   <213> Homo sapiens
<220>
   <221> misc_feature
   <222> (1)..(1)
   <223> biotinylated base
<220>
   <221> misc_feature
   <222> (40)..(40)
   <223> biotinylated base
<400> 41
   ctgcacacac cagttgagca ggtactggga gccaatattg 40
<210> 42
   <211> 40
   <212> DNA
   <213> Homo sapiens
<220>
   <221> misc_feature
   <222> (1)..(1)
   <223> biotinylated base
<220>
   <221> misc_feature
   <222> (40)..(40)
   <223> biotinylated base
<400> 42
   ccacttgata ggcactttgc ctccttctgc atggtattct 40
<210> 43
   <211> 30
   <212> DNA
   <213> Homo sapiens
<220>
   <221> misc_feature
   <222> (1)..(1)
   <223> biotinylated base
<220>
   <221> misc_feature
   <222> (30)..(30)
   <223> biotinylated base
<400> 43
   gctcacgcct gtaatcccag cactttggga 30
<210> 44
   <211> 30
   <212> DNA
   <213> Homo sapiens
<220>
   <221> misc_feature
   <222> (1)..(1)
   <223> GCTCACGCCTGTAATCCCAGCACTTTGGGA
<220>
   <221> misc_feature
   <222> (30)..(30)
   <223> GCTCACGCCTGTAATCCCAGCACTTTGGGA
<400> 44
   cggtgaaacc ccgtctctac taaaaataca 30
<210> 45
   <211> 29
   <212> DNA
   <213> Homo sapiens
<220>
   <221> misc_feature
   <222> (1)..(1)
   <223> GCTCACGCCTGTAATCCCAGCACTTTGGGA
<220>
   <221> misc_feature
   <222> (30)..(30)
   <223> GCTCACGCCTGTAATCCCAGCACTTTGGGA
<400> 45
   actcgggagg ctgaggcagg agaatggcg 29
<210> 46
   <211> 40
   <212> DNA
   <213> Homo sapiens
<400> 46
   cggatcacga ggtcaggaga tcgagaccat cctggctaac 40
<210> 47
   <211> 287
   <212> DNA
   <213> Homo sapiens
<400> 47
   ggccgggcgc ggtggctcac gcctgtaatc ccagcacttt gggaggccga ggcgggcgga     60
   tcacgaggtc aggagatcga gaccatcctg gctaacacgg tgaaaccccg tctctactaa    120
   aaatacaaaa aattagccgg gcgtggtggc gggcgcctgt agtcccagct actcgggagg    180
   ctgaggcagg agaatggcgt gaacccggga ggcggagctt gcagtgagcc gagatcgcgc    240
   cactgcactc cagcctgggc gacagagcga gactccgtct caaaaaa                  287

## Description

### TECHNICAL FIELD

The present invention relates to a method for detecting nucleic acid.

### BACKGROUND ART

Analysis of genetic information of various organisms has begun, and information on a number of genes including human genes and their base sequences, proteins encoded by the gene sequences, and sugar chains secondarily produced from these proteins, is being rapidly clarified. Functions of macromolecules such as genes, proteins and sugar chains whose sequences have been clarified can be investigated by various methods. Examples of methods for investigating nucleic acid mainly include Northern blotting and Southern blotting, by which relationships between various genes and expression of their biological functions can be investigated using complementarity between various nucleic acids. Examples of methods for investigating proteins include Western blotting, by which functions and expression of proteins can be investigated using reactions between proteins.

As a method for detecting nucleic acid, sandwich hybridization is known. Sandwich hybridization is carried out using a capture probe immobilized on a filter. The capture probe is complementary to a first portion of the target nucleic acid. In a stage of this method, the capture probe immobilized on a filter is exposed to the sample to be investigated for the target nucleic acid sequence, and further exposed to a labeled detection probe complementary to a second portion of the target nucleic acid. The second portion described above is different from (that is, does not overlap with) the portion of the target complementary to the first probe (Patent Documents 1 and 2, Non-patent Document 1). By this method, the labor required for immobilizing the sample on the filter can be eliminated, and a first probe suitable for the support can be selected.

### PRIOR ART DOCUMENTS

### [Patent Documents]

[Patent Document 1] US 4,486,539 B
[Patent Document 2] JP 7-75600 A
[Patent Document 3] Japanese Translated PCT Patent Application Laid-open No. 9-507121
[Patent Document 4] WO 99/47705

### [Non-patent Document]

[Non-patent Document 1] Sinikka Parkkinen et al., Journal of Medical Virology 20:279-288 (1986)

### SUMMARY OF THE INVENTION

### PROBLEMS TO BE SOLVED BY THE INVENTION

In sandwich hybridization, detection is generally carried out after preliminarily amplifying the sample by a nucleic acid amplification technique such as PCR. Amplification of the target nucleic acid increases the detection sensitivity, but, since contaminated DNA is also amplified, there is a concern of false positivity. Further, in cases where a plurality of genes are to be detected simultaneously, the number of primer sets increases as the number of genes increases, and therefore the quality control of the primer sets requires much labor.

On the other hand, a number methods for detecting a target nucleic acid without PCR amplification have been studied. Sensitization techniques, such as hybridization of a target nucleic acid with a capture probe followed by incorporation of a number of luminous bodies or fluorescent bodies using labeling substances or tag sequences as a base, have been devised, but these techniques require a special enzyme or complex reaction, or special support (e.g., optical planar waveguide) or special luminous body (a label that provides a signal detectable by evanescently excited luminescence) (Patent Document 4).

An object of the present invention is to provide a method for detecting a nucleic acid, in which the target nucleic acid can be detected with high sensitivity even in cases where the target nucleic acid is detected by sandwich hybridization using neither nucleic acid amplification nor a sensitization technique.

### MEANS FOR SOLVING THE PROBLEMS

As a result of intensive study, the present inventors discovered that, in sandwich hybridization, a target nucleic acid can be detected with high sensitivity by simultaneously hybridizing a plurality of detection probes that hybridize with different regions in the target nucleic acid, even in cases where neither nucleic acid amplification nor a sensitization technique is used, thereby completing the present invention.

That is, the present invention provides the following.
(1) A method for detecting a target nucleic acid, the method comprising the steps of:
   sequentially or simultaneously bringing a target nucleic acid or fragmentation product thereof, a plurality of detection probes, and a capture probe immobilized on a support, into contact with each other to hybridize the capture probe with the target nucleic acid or fragmentation product thereof and to hybridize the target nucleic acid or fragmentation product thereof with the plurality of detection probes, thereby binding the plurality of detection probes to the support through the capture probe and the target nucleic acid or fragmentation product thereof; and
   detecting the plurality of detection probes bound to the support.
(2) The method according to (1), wherein the step of sequentially or simultaneously bringing a target nucleic acid or fragmentation product thereof, a plurality of detection probes, and a capture probe immobilized on a support, into contact with each other is sequentially carried out by hybridizing the target nucleic acid or fragmentation product thereof with the plurality of detection probes and then hybridizing the target nucleic acid or fragmentation product thereof hybridized with the plurality of detection probes with the capture probe.
(3) The method according to (1) or (2), wherein the mode of the nucleic acid length of the target nucleic acid or fragmentation product thereof to be hybridized with the capture probe is within the range of 100 bases to 1500 bases.
(4) The method according to any one of (1) to (3), wherein, in the target nucleic acid or fragmentation product thereof to be hybridized with the capture probe, a plurality of detection probes are hybridized at distances of not more than 1500 bases from the binding position of the capture probe.
(5) The method according to any one of (1) to (3), wherein, in the target nucleic acid or fragmentation product thereof to be hybridized with the capture probe, a plurality of detection probes are hybridized at distances of not more than the mode of the nucleic acid length of the target nucleic acid or fragmentation product thereof from the binding position of the capture probe.
(6) The method according to any one of (1) to (5), wherein a fragmentation product of the target nucleic acid prepared by carrying out a fragmentation treatment of the target nucleic acid such that the mode of the nucleic acid length is within the range of 100 bases to 1500 bases is hybridized with the capture probe.
(7) The method according to any one of (1) to (6), wherein the target nucleic acid or fragmentation product thereof to be hybridized with the capture probe has not undergone amplification by a nucleic acid amplification method.
(8) The method according to any one of (1) to (7), wherein a human-derived sample containing the target nucleic acid is subjected to the detection method, the detection method further comprising a step of detecting at least one type of repetitive sequences present in the human genome as an internal standard, the repetitive sequences being contained in fragments of the human genome.
(9) The method according to (8), further comprising a step of fragmenting the human genome, wherein the repetitive sequences are contained in the fragmented human genome.
(10) The method according to (8) or (9), wherein the repetitive sequences are short interspersed nuclear elements.
(11) The method according to claim (10), wherein the short interspersed nuclear elements are Alu sequences.

### EFFECT OF THE INVENTION

By the present invention, a nucleic acid can be detected with high sensitivity even without using nucleic acid amplification such as PCR or a sensitization technique.

### BRIEF DESCRIPTION OF THE DRAWINGS

Fig. 1 is a schematic diagram for explaining the principle of the method of the present invention.
Fig. 2 is a diagram illustrating an example of nucleic acid length analysis.
Fig. 3 is a diagram schematically illustrating the positions of the capture probe and the detection probes in Example 1.
Fig. 4 is a diagram schematically illustrating the positions of the capture probe and the detection probes in Example 2.
Fig. 5 is a schematic diagram showing sandwich hybridization in mutation analysis.
Fig. 6 is a diagram schematically illustrating the positions of the k-ras mutation capture probe and the detection probes in Examples 3 and 4.
Fig. 7 is a diagram schematically illustrating the positions of the EGFR mutation capture probe and the detection probes in Examples 3 and 4.
Fig. 8 is a diagram schematically illustrating an embodiment in which the method of the present invention is carried out using Alu sequences as an internal standard in the human genome contained in a human-derived sample.

### BEST MODE FOR CARRYING OUT THE INVENTION

Examples of the target nucleic acid subjected to the detection method of the present invention include, but are not limited to, genes in pathogenic bacteria and viruses; causative genes for genetic diseases; and portions of such genes. Examples of specimens containing such a target nucleic acid include, but are not limited to, body fluids such as blood, serum, blood plasma, urine, stool, spinal fluid, saliva, swabs and tissue fluids; tissues; paraffin-embedded samples (FFPE) and sections thereof; and foods and beverages as well as dilutions thereof. The target nucleic acid as a test substance may be a sample nucleic acid extracted from blood or cells by a normal method, and DNA, RNA or the like extracted from the sample may be used. Examples of the DNA include, but are not limited to, chromosomal DNAs; viral DNAs; DNAs from bacteria, molds and the like; cDNAs obtained by reverse transcription of RNAs; and fragments as a part of these DNAs. Examples of the RNA include, but are not limited to, messenger RNAs, ribosomal RNAs, small RNAs, and fragments as a part of these RNAs. A chemically synthesized DNA or RNA may also be used as the target nucleic acid.

The sample nucleic acid may contain a nucleic acid component other than the target nucleic acid to be measured (non-target nucleic acid). Such a non-target nucleic acid may be removed in consideration of a difference in a property from the target nucleic acid, or may be used as a test substance without removal.

The target nucleic acid may be amplified by a nucleic acid amplification method such as PCR using the target nucleic acid as a template, and this can largely increase the measurement sensitivity. In cases where a nucleic acid amplification product is used as the target nucleic acid, the amplified nucleic acid can be labeled by performing the amplification in the presence of a nucleoside triphosphate labeled with a fluorescent dye or the like. However, by the method of the present invention, the target nucleic acid can be detected with sufficient sensitivity even without use of a nucleic acid amplification method. Further, since use of a nucleic amplification method causes problems such as false positivity and laborious operations, the present invention is especially useful in cases where it is applied to a target nucleic acid that has not undergone amplification by a nucleic acid amplification method, or a fragmentation product thereof.

The method of the present invention can be used for distinctive detection of the presence or absence of a target nucleic acid, genotype of a virus, species or strain of a bacterium, or species or strain of a mold; detection of an SNP (single nucleotide polymorphism); detection of a messenger RNA; detection of an miRNA; CGH; or detection of copy number variation, deletion/duplication/fusion of a genomic DNA sequence or deletion/duplication/fusion of a transcription product. Further, the present invention can also be applied to quantification of a target nucleic acid by measuring the intensity of a signal from a detection probe. Since quantification of a target nucleic acid is inevitably accompanied by detection of the target nucleic acid, the "detection method" of the present invention also includes cases accompanied by quantification.

Either the target nucleic *acid per se* or a fragmentation product of the target nucleic acid can be applied to the method of the present invention. In cases where the target nucleic acid is long (with a length of not less than 1500 bases, especially not less than 4000 bases), a fragmentation product having an appropriate length prepared by a fragmentation treatment is preferably applied as described later. The fragmentation product may be applied as it is to the method of the present invention without selection of a specific nucleic acid fragment from the nucleic acid fragments produced, and, by this, the detection sensitivity can be increased.

Examples of the method for cleaving the target nucleic acid for fragmentation include cleavage by ultrasonic irradiation, cleavage by an enzyme, cleavage by a restriction enzyme, use of a nebulizer, and cleavage by an acid or alkali. In cases of cleavage by ultrasonic irradiation, cleavage to a desired length is possible by controlling the output intensity and irradiation time for ultrasonic irradiation of the target nucleic acid.

The degree of fragmentation of the treated target nucleic acid can be analyzed by analysis methods such as electrophoresis described below. In cases where the ultrasonic treatment was found to be insufficient as a result of analysis, further ultrasonic treatment may be performed until a target nucleic acid having a desired property can be obtained. Examples of the ultrasonic processor include Acoustic Solubilizer (Covaris Inc.), Bioruptor (Tosho Denki) and Ultrasonic Homogenizer (Taitec Corporation, VP-050). In Acoustic Solubilizer S220, manufactured by Covaris Inc., cleavage to a desired length can be achieved by setting 4 parameters-Duty Factor, Peak incident power, Cycles per burst, and time. In cases where a nucleic acid having a mode of the nucleic acid length of 400 bases is desired, Duty Factor may be set to 10%; Peak incident power may be set to 140; Cycles per burst may be set to 200; and time may be set to 55. In cases where cleavage to a different length is desired, the cleavage may be carried out according to settings recommended by Covaris Inc.

In enzymatic cleavage, dSDNA shearase (Zymo Research), a restriction enzyme or the like may be used to obtain a nucleic acid fragment having a desired length by increasing/decreasing the incubation time. For example, in cases where dSDNA shearase is used, the cleavage may be carried out with the incubation time recommended by the manufacturer. For example, in cases where a nucleic acid having a mode of the nucleic acid length of 300 bases is desired, the manufacturer recommends incubation at 37°C for 40 minutes. Also in other DNA cleavage methods, the length of the cleavage fragment can be controlled by controlling treatment conditions.

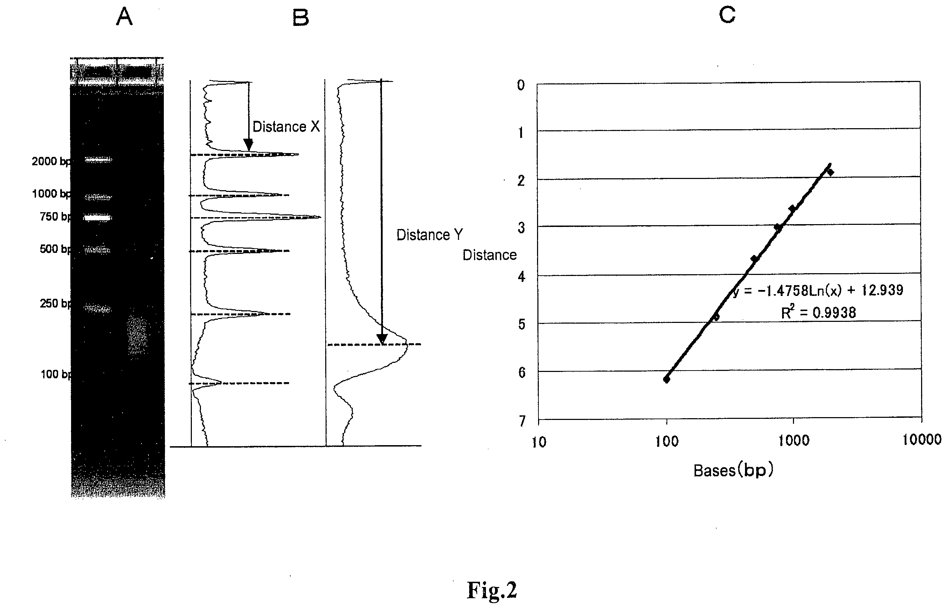

The fragmented target nucleic acid can be evaluated using as an index the mode of the nucleic acid length. The mode of the nucleic acid length means the peak top value obtained using an electrophoretic method such as agarose gel electrophoresis or Bioanalyzer (Agilent; DNA 7500 kit, RNA 6000 nano kit). The result of electrophoresis is shown as an electropherogram, and the highest position in the waveform is defined as the peak top. The value of the point where the perpendicular drawn from the peak top crosses the x-axis is defined as the mode of the nucleic acid length. One may refer to JP 4619202 B and the like for methods for analyzing the nucleic acid length. In cases where the analysis is carried out by agarose gel electrophoresis, a DNA ladder (e.g., product number 3415A manufactured by Takara Bio Inc.) may be subjected to the electrophoresis at the same time, and the mobilities of the ladder markers may be used as an index for measuring the peak top of the cleavage fragment. Fig. 2A is an agarose gel electropherogram of a ladder marker and a cleaved nucleic acid. This image is displayed as a waveform based on the brightness using image processing software such as NIH Image (NIH). Then, the distance of each ladder marker from the origin of electrophoresis is determined. In the case of the cleaved nucleic acid, the distance to the peak top, that is, the portion with the highest brightness, is determined. Based on the distances obtained for the ladder markers, a calibration curve and a regression equation as shown in Fig. 2C are obtained. By substituting the distance Y into the regression equation, the mode of the cleaved target nucleic acid can be determined. Based on the analysis as described above, the mode of the nucleic acid length of the cleaved target nucleic acid used in Fig. 2 is 158 bases.

Although the shape of the waveform is not limited, a sharp waveform produces a more preferred result than a broad waveform.

By the various methods described above, cleavage fragments having a desired mode of the nucleic acid length can be obtained. The mode of the nucleic acid length is preferably within the range of 100 bases to 1500 bases, more preferably within the range of 250 bases to 500 bases.

Also in cases where a test substance contaminated with non-target nucleic acid is used, the fragmentation treatment may be carried out, and the mode of the nucleic acid length can be evaluated in the same manner as described above.

The support may be a slide glass, membrane, beads or the like. Examples of the material of the support include, but are not limited to, inorganic materials such as glass, ceramic and silicon; and polymers such as polyethylene terephthalate, cellulose acetate, polycarbonate, polystyrene, polymethyl methacrylate and silicone rubber. Thus, in the present invention, supports that have been conventionally used in the art can be used, and a special support such as an optical planar waveguide does not need to be used. From the viewpoint of avoiding costliness and laboriousness, the support is preferably not an optical planar waveguide.

The capture probe means a substance that can directly and selectively bind to the target nucleic acid contained in the test sample. More specifically, in the method for detecting a target nucleic acid of the present invention, DNA, RNA, PNA or a nucleic acid derivative such as LNA (Locked Nucleic Acid) may be used. In cases of nucleic acid, the derivative herein means a chemically modified derivative, and examples of the chemically modified derivative include derivatives labeled with a fluorophore or the like; and derivatives containing a modified nucleotide (e.g., nucleotide containing a halogen or a group such as alkyl including methyl, alkoxy including methoxy, thio, or carboxymethyl; or nucleotide that has undergone, for example, reconstruction of a base, saturation of a double bond, deamination, or substitution of an oxygen molecule by a sulfur molecule).

A single-stranded nucleic acid having a specific base sequence serves as the capture probe of the present invention since it selectively hybridizes with a single-stranded nucleic acid having a base sequence complementary to the specific base sequence or to a part of the specific base sequence. The capture probe used in the present invention may be one commercially available or may be obtained from living cells or the like. An especially preferred capture probe is a nucleic acid. Among nucleic acids, nucleic acids called oligonucleic acids, which have lengths of not more than 200 bases, can be easily artificially synthesized by a synthesizer.

As the capture probe, those containing a sequence complementary to the target nucleic acid sequence can be employed, and any region may be selected. Its sequence preferably does not overlap with the sequence of the detection probe described below. Further, a plurality of types of capture probes that hybridize with different regions in the target nucleic acid may be used. However, since, in the present invention, a satisfactory sensitivity can be obtained even with a single type of capture probe, a single type of capture probe is preferably used for each target nucleic acid in view of simplicity.

In cases where the target nucleic acid is a double-stranded DNA, a sequence complementary to the Watson strand (sense strand) or the Crick strand (antisense strand) may be selected as the capture probe. As the sequences of the detection probe and the capture probe described below, sequences on the same strand are preferably selected.

In cases where a target nucleic acid contained in a sample nucleic acid is to be distinctively detected among different target nucleic acids, for example, in cases where the type of a virus with which a patient is infected is to be distinctively detected, it is preferred to select a highly specific sequence region among the nucleic acid sequences that may be contained in the sample nucleic acid. This means that, among all sequences contained in the sample nucleic acid, there is no sequence that is highly homologous to the sequence selected as the capture probe except for the above region.

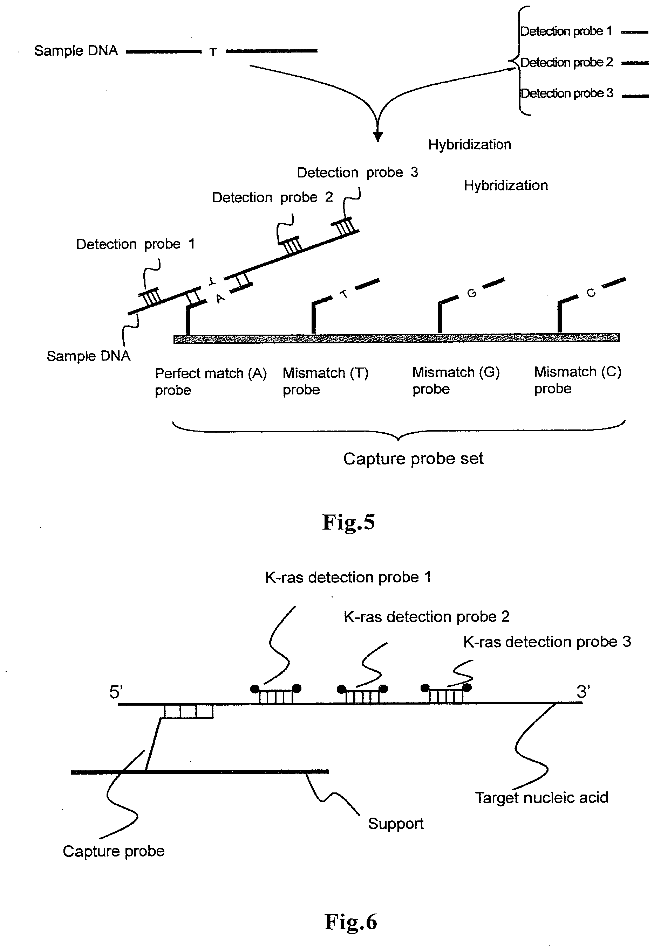

A capture probe that may be used for the detection of a single nucleotide polymorphism may be designed using a method proposed in Patent Document 3. More specifically, a base suspected of mutation is placed at the center of the capture probe, and 10 bases are added to each of the 5'-side and 3'-side of the base, to prepare a capture probe having a total length of 21 bases. A capture probe in which A is placed at the base position suspected of mutation, a capture probe in which T is placed at the base position suspected of mutation, a capture probe in which G is placed at the base position suspected of mutation, and a capture probe in which C is placed at the base position suspected of mutation, may be used as a capture probe set (Fig. 5). Further, in cases where a plurality of SNPs are to be detected, the sequences of the capture probe sets are more preferably selected such that they have similar Tm values. For example, the lengths of capture probe sequences may be controlled, or an artificial nucleic acid such as LNA may be used.

The homology (%) can be determined using a homology search program (e.g., BLAST or FASTA) conventionally used in the art, with default settings. In another aspect, the homology (%) can be determined by an arbitrary algorithm known in the art, such as the algorithm by Needleman et al. (1970) (J. Mol. Biol. 48:444-453), or Myers and Miller (CABIOS, 1988, 4:11-17). The algorithm by Needleman et al. is incorporated in the GAP program in the GCG software package (available at www.gcg.com), and the homology (%) can be determined by using, for example, the BLOSUM 62 matrix or PAM 250 matrix; gap weight of 16, 14, 12, 10, 8, 6 or 4; and length weight of 1, 2, 3, 4, 5 or 6. The algorithm by Myers and Miller is incorporated in the ALIGN program, which is a part of the GCG sequence alignment software package.

The detection probe means a substance that can directly bind to the target nucleic acid contained in the test sample. More specifically, in the method for detecting a target nucleic acid of the present invention, a nucleic acid derivative of DNA, RNA, PNA or LNA (Locked Nucleic Acid) may be used. In cases of nucleic acid, the derivative herein means a chemically modified derivative, and examples of the chemically modified derivative include derivatives labeled with a fluorophore or the like; and derivatives containing a modified nucleotide (e.g., nucleotide containing a halogen or a group such as alkyl including methyl, alkoxy including methoxy, thio, or carboxymethyl; or nucleotide that has undergone, for example, reconstruction of a base, saturation of a double bond, deamination, or substitution of an oxygen molecule by a sulfur molecule).

A single-stranded nucleic acid having a specific base sequence is included in the detection probe of the present invention since it selectively hybridizes with a single-stranded nucleic acid having a base sequence complementary to the specific base sequence or to a part of the specific base sequence. The detection probe used in the present invention may be one commercially available or may be obtained from living cells or the like. An especially preferred detection probe is a nucleic acid. Among nucleic acids, nucleic acids called oligonucleic acids, which have lengths of not more than 200 bases, can be easily artificially synthesized by a synthesizer.

In cases where the target nucleic acid is a double-stranded DNA, a sequence complementary to either the Watson strand or the Crick strand may be selected as the detection probe. As the sequence of the capture probe and the detection probe described above, sequences on the same strand are preferably selected.

The detection probe may contain a sequence complementary to the target nucleic acid sequence, and any region may be selected. Its sequence preferably does not overlap with the sequence of the capture probe described above. A sequence at a distance of not more than 1500 bases from the position of the capture probe is preferably selected.

The sequence of the detection probe preferably has a low homology to the sequence of the capture probe, and the homology is preferably not more than 80%. The sequence may be determined in consideration of the stringency during the hybridization.

The stringency during the hybridization is known to be a function of the temperature, salt concentration, chain length of the probe, GC content of the nucleotide sequence of the probe, and the concentration of the chaotropic agent in the hybridization buffer. Examples of stringent conditions that may be used include the conditions described in Sambrook, J. et al. (1998) Molecular Cloning: A Laboratory Manual (2nd ed.), Cold Spring Harbor Laboratory Press, New York. A stringent temperature condition is not less than about 30°C. Examples of other conditions include the hybridization time, concentration of the washing agent (e.g., SDS) and presence or absence of a carrier DNA. Various stringencies can be set by combining these conditions. Those skilled in the art can appropriately determine conditions for obtaining functions of the capture probe and detection probe provided for detection of a desired target nucleic acid.

In cases where a plurality of types of target nucleic acids contained in a sample nucleic acid are to be detected, a sequence having a homology of 100% among the target nucleic acids to be detected may be used as a common detection probe. The common detection probe may have a degenerate sequence in cases where the homology among the target nucleic acids to be detected is not 100%. For the degenerate sequence, one may refer to "Biological Experiment Illustrated - Truly Productive PCR", (1999), Shujunsha Co., Ltd., pp. 121 to 125.

A labeling substance may be bound to the detection probe. In cases where the detection probe is a nucleic acid, a labeling substance(s) may be bound to one or both of the 5'-end and the 3'-end. Further, a labeling substance may be introduced into the detection probe. The labeling substance may be bound by chemical reaction, enzymatic reaction or the like. The reaction is preferably carried out using chemical reaction. More preferably, a labeling substance is bound to the end(s) during chemical synthesis of the detection probe. The labeling substance may also be bound to the inside of the detection probe. For the binding of a labeling substance, chemical reaction may be used, and a biotin label may be inserted by a synthesizer. Biotin-TEG, Biotin-ON, Biotin-dT or the like may be inserted (https://www.operon.jp/index.php).

Examples of labeling substances that may be used in the present invention include known substances used for labeling, such as protein-binding substances, fluorescent dyes, phosphorescent dyes and radioisotopes. The labeling substance is preferably a protein-binding substance. Examples of the protein-binding substance include biotin. Biotin can bind to avidin or streptavidin. Avidin or streptavidin to which a fluorescent dye is bound, or avidin or streptavidin to which an enzyme such as alkaline phosphatase or horse radish peroxidase is bound may be used. In cases where alkaline phosphatase or horse radish peroxidase is used, its substrate is added, and reaction of the substrate with the enzyme results in occurrence of luminous reaction. The luminous reaction is detected using a plate reader, CCD camera or the like.

As described above, in the present invention, labels that have been conventionally used in the art may be employed, and the invention does not require use of a special label such as a label that provides a signal detectable by evanescently excited luminescence. From the viewpoint of avoiding costliness and laboriousness, the label is preferably not a label that provides a signal detectable by evanescently excited luminescence.

As the labeling substance, a fluorescent dye that can be simply measured and whose signal can be easily detected may be used. Specific examples of the fluorescent dye include known fluorescent dyes such as cyanine (Cyanine 2), aminomethylcoumarin, fluorescein, indocarbocyanine (Cyanine 3), Cyanine 3.5, tetramethylrhodamine, rhodamine red, Texas red, indocarbocyanine (Cyanine 5), Cyanine 5.5, Cyanine 7, Oyster, BODIPY dyes, and phycoerythrin.

Further, as the labeling substance, a luminescent semiconductor particle may be used. Examples of such a semiconductor particle include cadmium selenide (CdSe), cadmium telluride (CdTe), indium gallium phosphide (InGaP), chalcopyrite particles, and silicon (Si). The fluorescent dye can be detected with a fluorescence microscope, fluorescence scanner or the like.

The detected signal is compared with the noise in its vicinity. More specifically, the signal value obtained for the position where the capture probe is immobilized is compared with the signal value obtained for another position. The target nucleic acid is regarded as being detected in cases where the former value is higher than the latter value.

Examples of known methods for immobilizing a capture probe on a support include methods in which an oligo DNA is synthesized on the upper surface of the support, and methods in which an oligo DNA preliminarily synthesized is dropped onto the upper surface of the support and then immobilized. Examples of the former methods include a method by Ronald et al. (US 5705610 B), method by Michel et al. (US 6142266 B), and method by Francesco et al. (US 7037659 B). Since these methods use an organic solvent for the DNA synthesis reaction, the material of the carrier is preferably resistant to organic solvents. For example, a glass carrier having an irregular structure prepared using a method described in Japanese Translated PCT Patent Application Laid-open No. 10-503841 may be used. In particular, since, in the method by Francesco et al., the carrier is irradiated with light from the back side of the carrier to control DNA synthesis, the material of the carrier is preferably a translucent material. Examples of the latter methods include a method by Hirota et al. (JP 3922454 B) and use of a glass capillary. Examples of the glass capillary include, but are not limited to, self-made glass capillaries and commercially available products such as micropipettes (manufactured by Microsupport Co., Ltd., MP-005).

The preparation of DNA or RNA from living cells may be carried out by a known method. For example, the DNA can be extracted by a method by Blin et al. (Blin et al., Nucleic Acids Res. 3: 2303 (1976)) or the like, and the RNA can be extracted by a method by Favaloro et al. (Favaloro et. al., Methods Enzymol. 65: 718 (1980)) or the like. Further, as the nucleic acid to be immobilized, a linear or circular plasmid DNA, chromosomal DNA, DNA fragments prepared by cleavage of such a DNA with a restriction enzyme or by chemical cleavage, DNA synthesized *in vitro* by an enzyme or the like, or a chemically synthesized oligonucleotide may also be used.

Since the method of the present invention is a sandwich hybridization, the basic operation itself of the method is the same as that of the known sandwich hybridization. That is, a target nucleic acid or fragmentation product thereof, a plurality of detection probes, and a capture probe immobilized on a support, are sequentially or simultaneously brought into contact with each other to hybridize the capture probe with the target nucleic acid or fragmentation product thereof and to hybridize the target nucleic acid or fragmentation product thereof with the plurality of detection probes, thereby binding the plurality of detection probes to the support through the capture probe and the target nucleic acid or fragmentation product thereof; and the plurality of detection probes bound to the support are then detected. The step of sequentially or simultaneously bringing a target nucleic acid or fragmentation product thereof, a plurality of detection probes, and a capture probe immobilized on a support, into contact with each other may be carried out by: (1) first bringing the target nucleic acid or fragmentation product thereof into contact with the plurality of detection probes to allow hybridization, and then bringing the target nucleic acid or fragmentation product thereof hybridized with the plurality of detection probes into contact with the capture probe immobilized on the support to allow hybridization; (2) conversely, first bringing the target nucleic acid or fragmentation product thereof into contact with the capture probe immobilized on the support to allow hybridization, and then bringing the target nucleic acid or fragmentation product thereof hybridized with the capture probe into contact with the plurality of detection probes to allow hybridization; or (3) simultaneously bringing the target nucleic acid or fragmentation product thereof, the plurality of detection probes, and the capture probe immobilized on a support, into contact with each other to hybridize the capture probe with the target nucleic acid or fragmentation product thereof and to hybridize the target nucleic acid or fragmentation product thereof with the plurality of detection probes. Among these, the method (1) described above, in which the contacting is sequentially carried out, is preferred since it often results in a higher detection sensitivity.

Each hybridization step may be carried out in exactly the same manner as in conventional methods. The reaction temperature and time may be appropriately selected depending on the chain length of the nucleic acid to be hybridized. In cases of nucleic acid hybridization, the reaction is usually carried out at about 30°C to 70°C for 1 minute to 10 and several hours, and, in cases of immune reaction, the reaction is carried out at room temperature to about 40°C for about 1 minute to several hours.

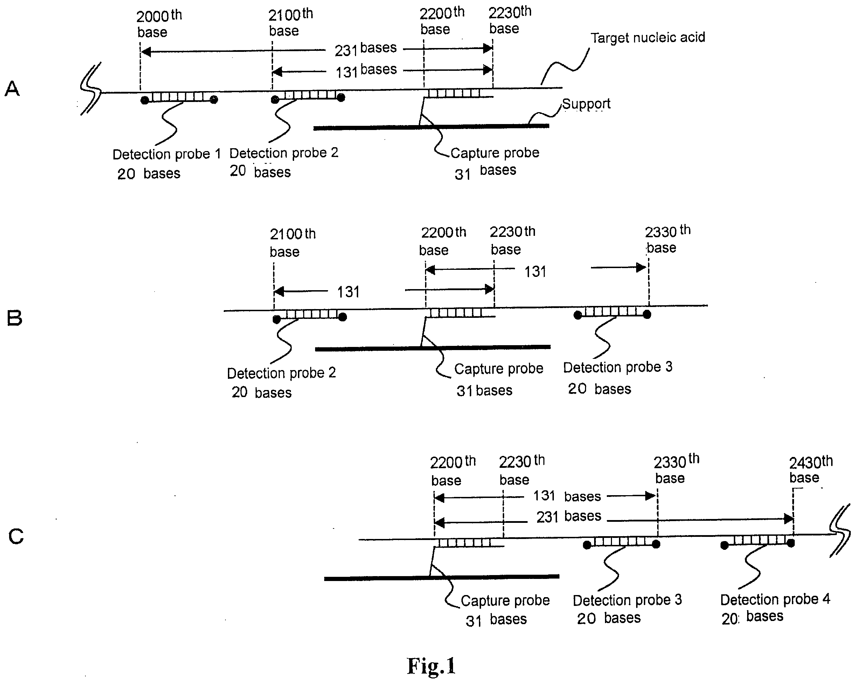

The concept of the present invention is described using Fig. 1. In this example, a target nucleic acid having a total length of 3000 bases is detected.

The capture probe may be designed for any part of the target nucleic acid. In this example, the region from the 2200th base to the 2230th base as counted from the beginning of the target nucleic acid was used as the sequence to which the capture probe is bound. Four detection probes were designed within the region in which the distance from the binding region of the capture probe is not more than 1500 bases. The detection probes and their sequence regions are as follows. Detection Probe 1, from the 2000th base to the 2019th base; Detection Probe 2, from the 2100th base to the 2119th base; Detection Probe 3, from the 2311th base to the 2330th base; and Detection Probe 4, from the 2411th base to the 2430th base.

The distance from the capture probe is determined by placing the binding position of the capture probe and the binding position of the detection probe on the target nucleic acid sequence, and counting the number of bases therebetween such that the most distal bases are counted as the end portions. An explanation is given using the example shown in Fig. 1B. The distance between Detection Probe 2, from the 2100th base to the 2119th base, and the capture probe, from the 2200th base to the 2230th base, is counted by regarding the positions of the most distal bases as the end portions. Therefore, the distance is 131 bases. The distance between Detection Probe 3, from the 2311th base to the 2330th base, and the capture probe, from the 2200th base to the 2230th base, is counted by regarding the positions of the most distal bases as the end portions. Therefore, the distance is 131 bases. According to such calculation, the distance of each of Detection Probes 1 and 4 from the capture probe is similarly 231 bases.

Subsequently, the target nucleic acid is cleaved such that the mode of the nucleic acid is 250 bases. Fig. 1A, Fig. 1B, and Fig. 1C are schematic diagrams illustrating hybridization of the target nucleic acid, Detection Probes 1, 2, 3 and/or 4, and the capture probe.

Since the target nucleic acid is cleaved at arbitrary positions, binding occurs in various modes as shown in Fig. 1.

Fig. 1A shows a mode of hybridization of a fragment produced by cleavage of the target nucleic acid at the 2231 st or a later base. Detection Probe 1 and Detection Probe 2 can bind to the target nucleic acid.

Fig. 1B shows a mode of hybridization of a fragment produced by cleavage of the target nucleic acid at a position before the 2100th base and a position after the 2330th base. Detection Probe 2 and Detection Probe 3 can bind to the target nucleic acid.

Fig. 1C shows a mode of hybridization of a fragment produced by cleavage of the target nucleic acid at a position before the 2200th base and a position after the 2430th base. Detection Probe 3 and Detection Probe 4 can bind to the target nucleic acid.

By providing a plurality of types of detection probes and cleaving the target nucleic acid such that the mode of the nucleic acid length is 250 bases, the detection can be carried out by one or more of the modes shown in Fig. 1.

On the other hand, in cases where only a single type of detection probe is provided, detection is impossible in one or more of the cases. For example, in cases where only Detection Probe 1 is used, detection is impossible in the cases of Fig. 1B and Fig. 1C. Therefore, the detection sensitivity is low.

In cases where the mode of the nucleic acid length is 150 bases, the detection probes that can bind to the target nucleic acid bound to the capture probe are Detection Probe 2 and Detection Probe 3. Therefore, the detection sensitivity is low.

Although Fig. 1 shows an example in which detection probes are designed in both sides of the capture probe, the detection probes may also be positioned only in the 3'-end side or only in the 5'-end side.

In general, in a method for detecting a nucleic acid such as the method of the present invention, an internal standard is detected at the same time for confirming whether or not the detection method itself is properly carried out. That is, when a target nucleic acid is not detected by the detection method, it is impossible to judge whether the target nucleic acid is absent in the test sample or the detection method was not properly carried out. Therefore, nucleic acid regions ubiquitously present in the test sample are used as an internal standard. In cases where this internal standard is detected, the detection method is judged to have been properly carried out, while in cases where the internal standard is not detected, the detection method is judged to have been improperly carried out. In cases where the test sample is derived from human, actin or globin is generally used as an internal standard. Also in the method of the present invention, the actin gene or globin gene may be used as an internal standard similarly to conventional methods, and, in particular, in cases where the target nucleic acid or its fragmentation product is amplified by PCR or the like, the same internal standards as in conventional methods may be used without any problem.

However, as described above, the detection method of the present invention is a method that exerts an excellent effect that enables detection of a target nucleic acid with sufficient sensitivity even without performing nucleic acid amplification. In cases where nucleic acid amplification is not carried out, the internal standard may not be detected even when the detection method is properly carried out since the actin gene and the globin gene are present in the genome as single-copy genes and hence the sensitivity may be insufficient.

In order to overcome this problem, in a preferred embodiment of the present invention, a sample derived from an animal such as human containing the target nucleic acid is subjected to the detection method described above, and at least one type of repetitive sequences present in the animal genome is detected as an internal standard. The repetitive sequences are contained in animal genome fragments.
The animal genome fragments may be produced by the fragmentation treatment of the animal genome, or may be naturally-occurring fragments. The preferred length of the genome fragment is the same as the preferred length of the target nucleic acid or fragmentation product thereof described above. Preferred examples of the animal include mammals such as human; pets including dog and cat; domestic animals including pig, cow, horse, sheep and goat; and laboratory animals including monkey, mouse and rat. Human is especially preferred, but other animals having such repetitive sequences may be used. Preferred examples of the repetitive sequences include retrotransposons, especially, short interspersed nuclear sequences (SINEs). In particular, Alu sequences, which are present in the human genome in 1,000,000 copies, are preferred. The Alu sequences per se are well-known sequences, and their base sequences are also well known (SEQ ID NO:47).

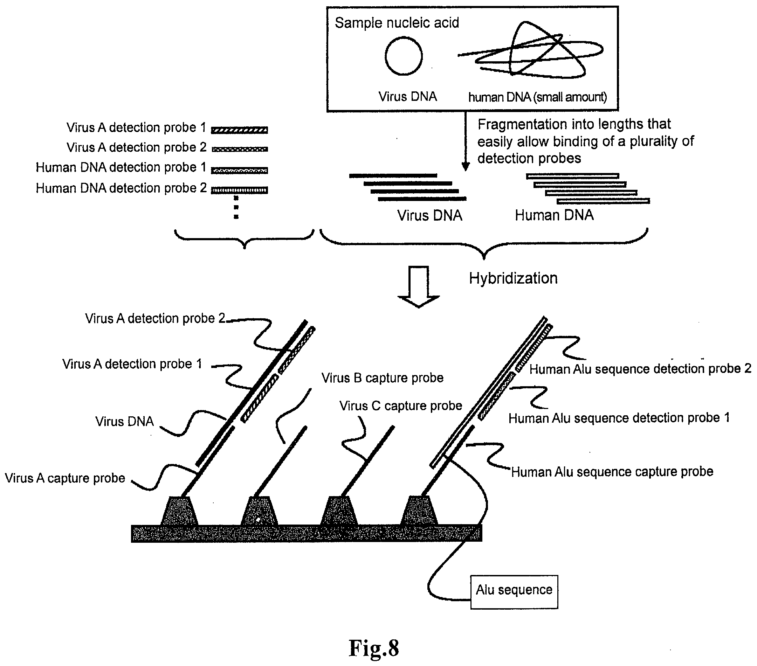

Fig. 8 is a schematic diagram for explanation of the principle of the method of the present invention in which a viral DNA is used as the target nucleic acid, and a human-derived sample containing the human genome is subjected to the detection method. In the method shown in Fig. 8, human Alu sequences are used as an internal standard. A human-Alu-sequence capture probe that captures human Alu sequences is immobilized on a support, and a plurality of types of human-Alu-sequence detection probes for detecting captured human DNA are hybridized with captured human Alu sequences, to detect the captured human Alu sequences. Here, the detection of animal DNA such as human Alu sequences can be carried out in the same manner as the in the method of detection of a target nucleic acid or fragmentation product thereof described above, and preferred conditions are also the same as described above.

### EXAMPLE 1

The present invention is described in more detail as an example in which the type of the virus with which a patient is infected is distinctively detected, by way of an Example for detection of human papillomavirus. However, the present invention is not limited by the Example below.

Human papillomavirus is known as a causative virus for cervical cancer. There are not less than 100 types of human papillomaviruses, and 13 types among these are highly malignant and may cause cervical cancer. In preventive medicine for cervical cancer, it is important to know the type of the virus with which the subject is infected, in order to determine the course of treatment. Identification of the type of human papillomavirus is carried out using a swab obtained from the cervix. Examples of known methods for the identification include the hybrid capture method and PCR method. By application of the present invention to detection of human papillomavirus, the type of the virus with which the patient is infected can be highly sensitively identified.

### (Design of Capture Probes and Detection Probes)

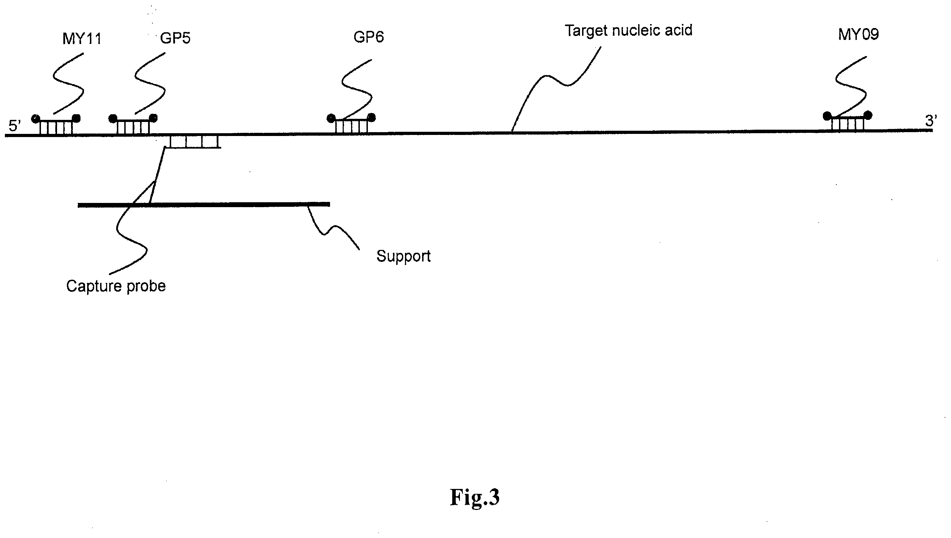

Studies on identification of the type of human papillomavirus have been carried out for a long time, and results of such studies can be utilized for selection of the capture probe. In the present example, capture probe sequences reported by a literature (J. Clin. Microbiol., 1995. pp. 901-905) were used (Table 1). In this literature, capture probes are designed such that the type can be identified using the sequence of the L1 gene region of human papillomavirus. The subject sequence is amplified using PCR primers MY11 and MY09 that are common among the types, and the amplified product is hybridized with capture probes immobilized on a filter, which capture probes specifically bind to the respective types, thereby achieving the detection. Therefore, the capture probes are designed to be positioned in almost the same regions in the L1 gene in all types. The present invention paid attention to the positions of the capture probes and the sequences of the common primers, and, by using the common primers as common detection probes, the distance from the capture probe was set almost the same among the types (Fig. 3, Table 2). As the capture probes having the sequences described above, synthetic DNAs modified with an amino group at the 5'-end were synthesized by Operon Biotechnologies, Inc. As the detection probes, those modified with biotin at the 3'-end and the 5'-end were synthesized by Operon Biotechnologies, Inc.

**[Table 1]**

| name | SEQ ID NO | Probe sequence 5'→3' |
|---|---|---|
| Probe 6 | SEQ ID NO:1 | ATCCGTAACTACATCTTCCACATACACCAA |
| Probe 11 | SEQ ID NO:2 | ATCTGTGTCTAAATCTGCTACATACACTAA |
| Probe 16 | SEQ ID NO:3 | GTCATTATGTGCTGCCATATCTACTTCAGA |
| Probe 18 | SEQ ID NO:4 | TGCTTCTACACAGTCTCCTGTACCTGGGCA |
| Probe 31 | SEQ ID NO:5 | TGTTTGTGCTGCAATTGCAAACAGTGATAC |
| Probe 33 | SEQ ID NO:6 | TTTATGCACACAAGTAACTAGTGACAGTAC |
| Probe 34 | SEQ ID NO:7 | TACACAATCCACAAGTACAAATGCACCATA |
| Probe 35 | SEQ ID NO:8 | GTCTGTGTGTTCTGCTGTGTCTTCTAGTGA |
| Probe 39 | SEQ ID NO:9 | TCTACCTCTATAGAGTCTTCCATACCTTCT |
| Probe 40 | SEQ ID NO:10 | GCTGCCACACAGTCCCCCACACCAACCCCA |
| Probe 42 | SEQ ID NO:11 | CTGCAACATCTGGTGATACATATACAGCTG |
| Probe 43 | SEQ ID NO:12 | TCTACTGACCCTACTGTGCCCAGTACATAT |
| Probe 44 | SEQ ID NO:13 | GCCACTACACAGTCCCCTCCGTCTACATAT |
| Probe 45 | SEQ ID NO:14 | ACACAAAATCCTGTGCCAAGTACATATGAC |
| Probe 51 | SEQ ID NO:15 | AGCACTGCCACTGCTGCGGTTTCCCCAACA |
| Probe 52 | SEQ ID NO:16 | TGCTGAGGTTAAAAAGGAAAGCACATATAA |
| Probe 54 | SEQ ID NO:17 | TACAGCATCCACGCAGGATAGCTTTAATAA |
| Probe 56 | SEQ ID NO:18 | GTACTGCTACAGAACAGTTAAGTAAATATG |
| Probe 58 | SEQ ID NO:19 | ATTATGCACTGAAGTAACTAAGGAAGGTAC |

**[Table 2]**

| Detection probe name | SEQ ID NO | Sequence 5'→3' | Distance from the capture probe |
|---|---|---|---|
| MY11 | SEQ ID NO:20 | GCMCAGGGWCATAAYAATGG | 50 |
| GP5 | SEQ ID NO:21 | GAAAAATAAACTGTAAATCATATTC | 10 |
| GP6 | SEQ ID NO:22 | TTTGTTACTGTGGTAGATACTAC | 60 |
| MY09 | SEQ ID NO:23 | GATCAGTWTCCYYTDGGACG | 340 |

### (Preparation of DNA Chip)

On a substrate of a DNA chip "3D-Gene" (registered trademark) (http://www.3d-gene.com/en/products/pro_009.html), all capture probes in Table 1 were immobilized to prepare a DNA chip. The details are as follows.

### (Preparation of DNA-immobilized Carrier)

Using a known method, the LIGA (Lithographie Galvanoformung Abformung) process, a mold for injection molding was prepared, and injection molding was carried out to obtain a PMMA carrier having the shape described below. Carbon black (Mitsubishi Chemical Corporation, #3050B) was contained in the PMMA at a ratio of 1 wt%, and the carrier had a black color. In terms of the shape of the carrier, the size was 76 mm in length, 26 mm in width, and 1 mm in thickness, and the surface of the carrier was flat except for the central portion. At the center of the carrier, a recess having a diameter of 10 mm and a depth of 0.2 mm was provided, and 64 (8×8) protruded portions each having a diameter of 0.2 mm and a height of 0.2 mm were provided in the recess. The pitch between the protruded portions (distance from the center of a protruded portion to the center of an adjacent protruded portion) in the irregular area was 0.6 mm.

### (Immobilization of Probe DNAs)

The capture probe DNAs in Table 1 were dissolved in pure water at a concentration of 0.3 nmol/µL to provide stock solutions. For spotting on the carrier, the final concentration of each probe DNA was adjusted to 0.03 nmol/µL with PBS (prepared by dissolving 8 g of NaCl, 2.9 g of Na₂HPO₄-12H₂O, 0.2 g of KCl, and 0.2 g of KH₂PO₄ in pure water to provide 1 L of a solution, and then adding hydrochloric acid to the solution for pH adjustment; pH 5.5), and 1-ethyl-3-(3-dimethylaminopropyl)carbodiimide (EDC) was added thereto at a final concentration of 50 mg/mL for condensation of carboxylic acid on the carrier surface with the amino group at the end of the probe DNA. Thereafter, the mixed solution was spotted on the upper surface of a protruded portion with a glass capillary. Subsequently, the carrier was placed in a tightly sealed plastic container, and incubated at 37°C at a humidity of 100% for about 20 hours, followed by washing with pure waster. After immobilization of the capture probe DNAs, a cover was attached to the central portion of the DNA chip, and zirconia beads were sealed between the DNA chip and the cover for stirring the solution during hybridization reaction.

### (Preparation of Sample DNA)

For the sample DNA, a recombinant plasmid pHPV16, which contains a cloned genomic DNA of human papillomavirus, was purchased from Health Science Research Resources Bank and used. The total length of pHPV16 was 16,600 base pairs. If the molecular weight of one base pair is regarded as 680, 1 µg corresponds to 89 nmol.

The method for preparing the sample DNA was as follows. By an ultrasonic treatment (Covaris Inc., s220), 1 µg of pHPV16 was fragmented. The fragmentation treatment was carried out under conditions where fragments having a length of 100 bases, 150 bases, 250 bases, 400 bases, 1500 bases or 4000 bases can be obtained according to the method recommended by the manufacturer. The lengths of fragments were evaluated using a Bioanalyzer (Agilent). As a result, the peak top values of the fragmented nucleic acids obtained under those treatment conditions were 100 bases, 150 bases, 250 bases, 400 bases, 1500 bases and 4000 bases, respectively. The concentration of the cleaved sample DNA was measured using NanoDrop (Thermo Fisher Scientific K.K., ND-1000) to determine the nucleic acid concentration. In consideration of the nucleic acid concentration, the cleaved DNA contained in each solution after cleavage was diluted to 1 amol/µL using 1×hybridization solution to provide a sample DNA.

### (Preparation of Detection Probe Solutions)

Each detection probe was diluted with sterile water to a concentration of 100 fmol/µL. Also in cases where a mixture of a plurality of detection probes is used, the dilution was carried out with sterile water such that the concentration of each detection probe was 100 fmol/µL.

### (Hybridization)

To 5 µL of the sample DNA, 1 µL of the detection probe dilution was added, and the resulting mixture was heated using a thermal cycler at 95°C for 5 minutes. Thereafter, the solution was left to stand on the bench for 2 minutes to allow the solution to cool to room temperature. To this solution, 35 µL of 1×hybridization solution (1 wt% BSA (bovine serum albumin), 5×SSC, 1 wt% SDS (sodium dodecyl sulfate), 50 ng/ml salmon sperm DNA solution, 5 wt% dextran sulfate sodium, 30% formamide) was added to provide a hybridization solution. The whole solution was injected into the DNA chip, and the DNA chip was placed in an incubator heated at 32°C. Hybridization was carried out according to the standard protocol for "3D-Gene" with rotary shaking at 250 rpm at 32°C for 2 hours. Thereafter, the DNA chip was washed for 5 minutes with a washing liquid (0.5×SSC, 0.1 wt% SDS (sodium dodecyl sulfate)) heated at 30°C, and dried using a spin dryer (Wakenyaku Co., Ltd.). A solution prepared by adding streptavidin-phycoerythrin (ProZyme, Inc.) to a staining solution (50 ng/µl streptavidin-phycoerythrin, 100 mM MES, 1 M NaCl, 0.05 wt% Tween 20, 2 mg/ml BSA (bovine serum albumin)) was provided, and the solution was dropped on the DNA chip. The DNA chip was then incubated at 35°C for 5 minutes. After washing the DNA chip for 5 minutes with a washing liquid (6×SSPE, 0.01 wt% Tween 20) heated at 30°C, the DNA chip was dried using a spin dryer (Wakenyaku Co., Ltd.). The DNA chip after staining was subjected to detection of fluorescence signals using a DNA chip scanner (Toray Industries, Inc.). In terms of settings of the scanner, the laser output was set to 100%, and the photomultiplier voltage was set to 70%.

The detection results are shown in Table 3. From the results of detection with the mixture of 4 types of detection probes, it can be seen that, compared to the uncleaved sample and the sample cleaved into 4000-base fragments, the samples cleaved into fragments having lengths of not more than 1500 bases showed predominantly stronger detection signals. In particular, it can be seen that the sensitivity was highest in the 250-base sample and the 500-base sample, and that the sensitivity began to decrease by further cleavage.

**[Table 3]**

| | | Signal value | | | | | | |
|---|---|---|---|---|---|---|---|---|
| Detection probe used | Distance between the capture probe and the detection probe (bases) | Mode of the DNA length (bases) | | | | | | |
| | | 100 | 150 | 250 | 500 | 1500 | 4000 | 8000 |
| i) 4 types GP6+GP5+MY11+MY09 | - | 2015 | 6246 | 14044 | 13005 | 7388 | 516 | 132 |
| ii) GP6 | 50 | 753 | 2337 | 5273 | 4900 | 3565 | 205 | 96 |
| iii) GP5 | 10 | 433 | 1201 | 3092 | 2800 | 1839 | 179 | 80 |
| iv) MY11 | 60 | 98 | 205 | 403 | 350 | 160 | 108 | 96 |
| v) MY09 | 340 | 0 | 0 | 204 | 800 | 1090 | 197 | 85 |
| vii) Sum of the signal values detected using the individual detection probes (ii+iii+iv+v) | - | 1284 | 3743 | 8972 | 8850 | 6654 | 689 | 357 |

In terms of the results of detection obtained by using the detection probes individually, GP5 and GP6 showed tendencies similar to the mixture of 4 types described above. Although MY11 also showed a similar tendency, its signal intensity was lower than those of GP5 and GP6, possibly due to a low degree of binding of the detection probe. MY09 is a detection probe designed for a position that is 340 bases distant from the capture probe, and, in cases where the DNA sample is cleaved into fragments with DNA lengths of not more than 250 bases, there is no region where the probe can bind to the target nucleic acid. Therefore, MY09 was expected to be incapable of functioning as a detection probe. Actually, in the Example, signals could be obtained for the 500-base sample and the 1500-base sample, but no signal was obtained for the 250-base and shorter samples.

It can be easily assumed that the sum of the signal intensities obtained by the individual detection probes is equivalent to the signal intensity obtained using the mixture of 4 types of detection probes. The sum of the signal intensities obtained by the individual detection probes is shown in the last line in Table 3. Compared to this, the signal intensity obtained using the mixture of 4 types showed much higher values in the cases where the target nucleic acid was cleaved into fragments of not more than 1500 bases. This was assumed to be due to enhancement of hybridization between the capture probe and the target nucleic acid by binding of the plurality of detection probes.

### EXAMPLE 2

The present invention is described in more detail by way of another embodiment of detection of human papillomavirus. However, the present invention is not limited by the Example below.

### (Design of Capture Probes and Detection Probes)

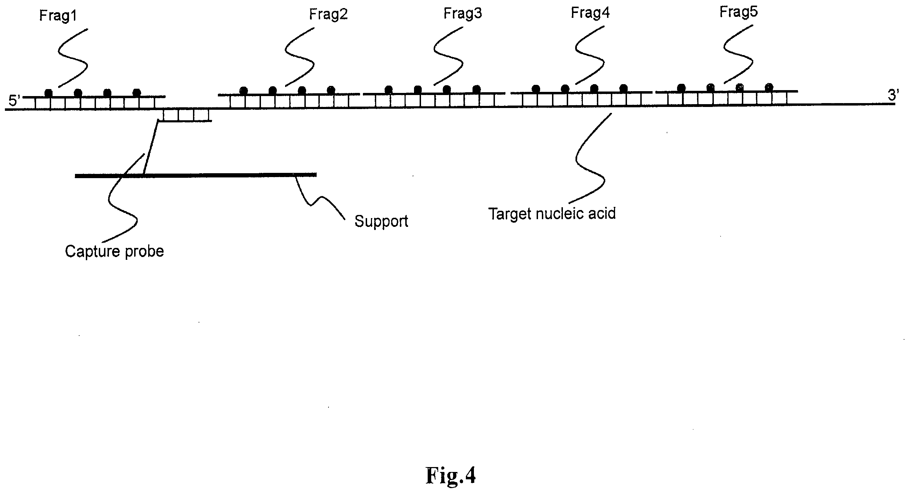

Similarly to Example 1, sequences reported in the literature (J. Clin. Microbiol., 1995. pp. 901-905, Table 1) were employed for capture probes. In general, the binding strength in nucleic acid hybridization increases as the length of the nucleic acid increases. In view of this, as detection probes, sequences each having a length of 80 to 86 bases were provided for 5 regions located upstream and downstream of the capture probe as shown in Fig. 4 and Table 4.

**[Table 4]**

| Detection probe name | SEQ ID NO | Sequence 5'→3' | Distance from the capture probe |
|---|---|---|---|
| Frag1 | SEQ ID NO:24 | gcacagggc[Bio-ON]acaataatggcatttgttg[Bio-ON] ggtaaccaactatttgttac[Bio-ON] gttgttgatactacacgca[Bio-ON]tacaaatat | 110 |
| Frag2 | SEQ ID NO:25 | aactacata[Bio-ON] aaaaatactaactttaagg[Bio-ON]gtacctacgacatggggaggaatatg[Bio-ON] tttacagtttatttttcaa[Bio-ON]tgtgcaaaa | 116 |
| Frag3 | SEQ ID NO:26 | taaccttaa[Bio-ON]tgcagacgttatgacatac[Bio-ON] tacattctatgaattccac[Bio-ON] attttggaggactggaatt[Bio-ON]tggtctacaacctc cc | 202 |
| Frag4 | SEQ ID NO:27 | ccaggaggc[Bio-ON]cactagaagatacttatag[Bio-ON] tttgtaacatcccaggcaattgcttg[Bio-ON] caaaaacatacacctccag[Bio-ON]acctaaaga | 288 |
| Frag5 | SEQ ID NO:28 | agatcccct[Bio-ON]aaaaaatacactttttggg[Bio-ON] agtaaatttaaaggaaaagttttc[Bio-ON] gcagacctagatcagtttc[Bio-ON]tttaggacg | 372 |

| | | | |
|---|---|---|---|
| Bio-on: biotin-on label | | | |

As the capture probes having the sequences described above, synthetic DNAs modified with an amino group at the 5'-end were synthesized by Operon Biotechnologies, Inc. As the detection probes, those internally labeled with biotin were synthesized by Operon Biotechnologies, Inc. (Table 4).

### (Preparation of DNA Chip)

A DNA chip on which all capture probes in Table 1 are immobilized was prepared. The method of preparing the DNA chip was the same as in Example 1.

### (Preparation of Sample DNA)

For the sample DNA, a recombinant plasmid pHPV16, which contains a cloned genomic DNA of human papillomavirus, was purchased from Health Science Research Resources Bank and used. The total length of pHPV16 was 16,600 base pairs. If the molecular weight of one base pair is regarded as 680, 1 µg corresponds to 89 nmol.

The method for preparing the sample DNA was as follows. By an ultrasonic treatment (Covaris Inc., s220), 5 µg of pHPV16 was fragmented. The fragmentation treatment was carried out under conditions where fragments having a length of 150 bases or 250 bases can be obtained according to the method recommended by the manufacturer. The lengths of fragments were evaluated using a Bioanalyzer (Agilent). As a result, the peak top values of the fragmented nucleic acids obtained under those treatment conditions were 150 bases and 250 bases, respectively. The concentration of the cleaved sample DNA was measured using NanoDrop (Thermo Fisher Scientific K.K., ND-1000) to determine the nucleic acid concentration. In consideration of the nucleic acid concentration, the cleaved DNA contained in each solution after cleavage was diluted to 1 amol/µL using 1×hybridization solution to provide a sample DNA.

### (Preparation of Detection Probe Solutions)

Each detection probe was diluted with sterile water to a concentration of 100 fmol/µL. Also in cases where a mixture of a plurality of detection probes is used, the dilution was carried out with sterile water such that the concentration of each detection probe was 100 fmol/µL.

### (Hybridization)

To 5 µL of the sample DNA, 1 µL of the detection probe dilution was added, and the resulting mixture was heated using a thermal cycler at 95°C for 5 minutes. Thereafter, the solution was left to stand on the bench for 2 minutes to allow the solution to cool to room temperature. To this solution, 35 µL of a hybridization solution (1 wt% BSA (bovine serum albumin), 5×SSC, 1 wt% SDS (sodium dodecyl sulfate), 50 ng/ml salmon sperm DNA solution, 5 wt% dextran sulfate sodium, 30% formamide) was added to provide a hybridization solution. The whole solution was injected into the DNA chip, and the DNA chip was placed in an incubator heated at 32°C. Hybridization was carried out according to the standard protocol for "3D-Gene" with rotary shaking at 250 rpm at 32°C for 2 hours. Thereafter, the DNA chip was washed for 5 minutes with a washing liquid (0.5×SSC, 0.1 wt% SDS (sodium dodecyl sulfate)) heated at 30°C, and dried using a spin dryer (Wakenyaku Co., Ltd.). A solution prepared by adding streptavidin-phycoerythrin (ProZyme, Inc.) to a staining solution (50 ng/µl streptavidin-phycoerythrin, 100 mM MES, 1 M NaCl, 0.05 wt% Tween 20, 2 mg/ml BSA (bovine serum albumin)) was provided, and the solution was dropped on the DNA chip. The DNA chip was then incubated at 35°C for 5 minutes. After washing the DNA chip for 5 minutes with a washing liquid (6×SSPE, 0.01 wt% Tween 20) heated at 30°C, the DNA chip was dried using a spin dryer (Wakenyaku Co., Ltd.). The DNA chip after staining was subjected to detection of fluorescence signals using a DNA chip scanner (Toray Industries, Inc.). In terms of settings of the scanner, the laser output was set to 100%, and the photomultiplier voltage was set to 70%.

The detection results are shown in Table 5. As a result of detection with 5 types of detection probes that were mixed together (mixture of 5 types), the signal intensity for the 150-base sample was stronger than the signal intensity for the 250-base sample. While 2 detection probes are effective in the case where the sample is cleaved into 150-base fragments, 3 detection probes are effective in the case where the sample is cleaved into 250-base fragments. It can be said that the difference in the signal intensity was due to the difference in the number of detection probes that could be bound. When the result obtained for the target nucleic acid cleaved into 250-base fragments is compared between the mixture of 4 types and the mixture of 5 types, the mixture of 5 types showed a stronger signal intensity. While 2 detection probes are effective in the case where the mixture of 4 types is used, 3 detection probes are effective in the case where the mixture of 5 types is used. Therefore, it can be said that the difference in the signal intensity was due to the difference in the number of detection probes that could be bound.

**[Table 5]**

| Nucleic acid length | Detection probes added | Effective detection probes | Detected signal |
|---|---|---|---|
| 150 bases | Mixture of 5 types (Detection Probes 1, 2, 3, 4, 5) | Detection Probes 1, 2 | 17,500 |
| 250 bases | Mixture of 4 types (Detection Probes 2, 3, 4, 5) | Detection Probes 2, 3 | 15,000 |
| 250 bases | Mixture of 5 types (Detection Probes 1, 2, 3, 4, 5) | Detection Probes 1, 2, 3 | 30,000 |

Thus, by providing a plurality of detection probes and performing cleavage of the target nucleic acid in consideration of the binding regions of the detection probes, an especially sufficient signal strength can be obtained. In cases where a mixture of 5 types of detection probes is used, the target nucleic acid is preferably cleaved into fragments of about 400 bases (± about 50 bases), more preferably cleaved into fragments of 400 bases.

### EXAMPLE 3

The present invention is described in more detail by way of an Example for detection of SNPs (single nucleotide polymorphisms). However, the present invention is not limited by the Example below. Since the response rate of cancer patients treated with an anticancer agent is known to be influenced by the presence/absence of mutations in the EGFR gene and the k-ras gene, the presence/absence of such mutations is used as a basis for judging whether the anticancer agent should be administered. In existing techniques, the mutations are detected by various methods after performing PCR amplification.

### (Design of Capture Probes and Detection Probes)

In the capture probe for detecting an SNP, the SNP site is placed at the center of the probe. More specifically, the 10 bases adjacent to the SNP site in each of the 5'-side and 3'-side are included in the capture probe sequence. In cases of detection of an SNP, a combination of capture probes corresponding to all bases at the SNP site are provided, and used as a capture probe set. More specifically, in cases of an SNP site where the wild type is G and the mutant type is A, capture probes each having a length of 21 bases containing the 10 bases adjacent to the G or A in each of the 5'-side and 3'-side are used. Further, for comparison, capture probes each having a length of 21 bases in which the central portion is T or C and the 10 bases adjacent to this portion in each of the 5'-side and 3'-side are contained are provided. These 4 sequences are used as the capture probe set. In this manner, a capture probe set for k-ras mutations Gly12Ser, Gly12Arg, and Gly12Cys; and a capture probe set for an EGFR mutation T790M; were provided (Table 6).

**[Table 6]**

| Probe set | Capture probe name | SEQ ID NO | **Mutation** | **Seqence (5'→3')** |
|---|---|---|---|---|
| K-ras capture probe set | K-ras capture probe, wild type | SEQ ID NO:29 | Wild type | **Agttggagctggtggcgtagg** |
| | K-ras capture probe, Gly12Ser | SEQ ID NO:30 | Gly12Ser | **Agttggagctagtggcgtagg** |
| | K-ras capture probe, Gly12Arg | SEQ ID NO:31 | Gly12Arg | **agttggagctcgtggcgtagg** |
| | K-ras capture probe, Gly12Cys | SEQ ID NO:32 | Gly12Cys | **agttggagcttgtggcgtagg** |
| EGFR capture probe set | EGFR capture probe, wild type | SEQ ID NO:33 | Wild type | **caactcatcacgcagctcatg** |
| | EGFR capture probe, T790M | SEQ ID NO:34 | T790M | **caactcatcatgcagctcatg** |
| | EGFRCapture probecontrol (a) | SEQ ID NO:35 | Control (a) | **caactcatcaagcagctcatg** |
| | EGFRCapture probecontrol (g) | SEQ ID NO:36 | Control (g) | **caactcatcaggcagctcatg** |

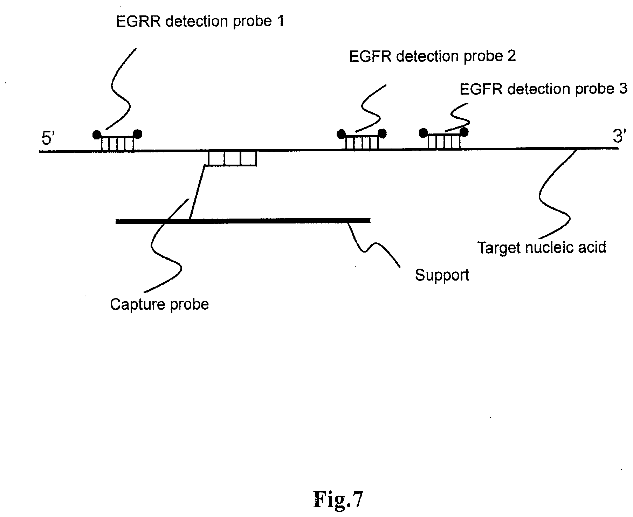

Detection probes were designed at the positions shown in Fig. 6 and Fig. 7 in consideration of their distances from the capture probes designed. The sequences of the detection probes and the distances of the detection probes from the capture probes are shown in Table 7 and Table 8.

**[Table 7]**

| Sequences of K-RAS detection probes and their distances from the capture probe (bases) | | | |
|---|---|---|---|
| Detection probe name | SEQ ID NO | Sequence 5'→3' | Distance from the capture probe (bases) |
| K-ras detection probe 1 | SEQ ID NO:37 | GATCATATTCGTCCACAAAATGATTCTGAATTAGCTGTAT | 75 |
| K-ras detection probe 2 | SEQ ID NO:38 | CCAAGAGACAGGTTTCTCCATCAATTACTACTTGCTTCCT | 135 |
| K-ras detection probe 3 | SEQ ID NO:39 | TCCTCATGTACTGGTCCCTCATTGCACTGTACTCCTCTTG | 191 |

**[Table 8]**

| Sequences of EGFR detection probes and their distances from the capture probe (bases) | | | |
|---|---|---|---|
| | SEQ ID NO | Sequence 5'→3' | Distance from the capture probe (bases) |
| EGFR detection probe 1 | SEQ ID NO:40 | AATTTTAACTTTCTCACCTTCTGGGATCCAGAGTCCCTTA | 198 |
| EGFR detection probe 2 | SEQ ID NO:41 | CTGCACACACCAGTTGAGCAGGTACTGGGAGCCAATATTG | 101 |
| EGFR detection probe 3 | SEQ ID NO:42 | CCACTTGATAGGCACTTTGCCTCCTTCTGCATGGTATTCT | 281 |

As the capture probes having the sequences described above, synthetic DNAs modified with an amino group at the 5'-end were synthesized by Operon Biotechnologies, Inc. As the detection probes, those labeled with biotin at the 3'-end and the 5'-end were synthesized by Operon Biotechnologies, Inc.

### (Preparation of DNA Chip)

A DNA chip on which all capture probes in Table 6 are immobilized was prepared. The method of preparing the DNA chip was the same as in Example 1.

### (Preparation of Sample DNA)

For the sample DNA, DNA extracted from A549 cells was used. A549 cells are cultured cells derived from a lung cancer tissue, and known to have the Gly12Ser mutation. By an ultrasonic treatment (Covaris Inc., s220), 5 µg of DNA extracted from A549 cells was fragmented. The fragmentation treatment was carried out under the conditions for the method recommended by the manufacturer. The lengths of fragments were evaluated using a Bioanalyzer (Agilent). As a result, the mode of the nucleic acid length was 750 bases. The whole cleaved DNA was used as the sample DNA.

### (Preparation of Detection Probe Solution)

The detection probes were mixed together, and diluted with sterile water such that the concentration of each detection probe was 100 fmol/µL.

### (Hybridization)

To 5 µL of the sample DNA, 1 µL of the detection probe dilution was added, and the resulting mixture was heated using a thermal cycler at 95°C for 5 minutes. Thereafter, the solution was left to stand on the bench for 2 minutes to allow the solution to cool to room temperature. To this solution, 35 µL of a hybridization solution (1 wt% BSA (bovine serum albumin), 5×SSC, 0.1 wt% SDS (sodium dodecyl sulfate), 0.01 wt% salmon sperm DNA solution) was added to provide a hybridization solution. The whole solution was injected into the DNA chip, and the DNA chip was placed in an incubator heated at 32°C. Hybridization was carried out according to the standard protocol for "3D-Gene" with rotary shaking at 250 rpm at 32°C for 2 hours. Thereafter, the DNA chip was washed for 5 minutes with a washing liquid (0.5×SSC, 0.1 wt% SDS (sodium dodecyl sulfate)) heated at 30°C, and dried using a spin dryer (Wakenyaku Co., Ltd.). A solution prepared by adding streptavidin-phycoerythrin (ProZyme, Inc.) to a staining solution (50 ng/µl streptavidin-phycoerythrin, 100 mM MES, 1 M NaCl, 0.05 wt% Tween 20, 2 mg/ml BSA (bovine serum albumin)) was provided, and the solution was dropped on the DNA chip. The DNA chip was then incubated at 35°C for 5 minutes. After washing the DNA chip for 5 minutes with a washing liquid (6×SSPE, 0.01 wt% Tween 20) heated at 30°C, the DNA chip was dried using a spin dryer (Wakenyaku Co., Ltd.). The DNA chip after staining was subjected to detection of fluorescence signals using a DNA chip scanner (Toray Industries, Inc.). In terms of settings of the scanner, the laser output was set to 100%, and the photomultiplier voltage was set to 70%.

**[Table 9]**

| | Mutation | Signal intensity | Probe with the strongest signal in the capture probe set |
|---|---|---|---|
| EGFR capture probe set | Control (g) | 935 | |
| | Control (a) | 1086 | |
| | T790M | 986 | |
| | Wild type | 1382 | O |
| K-ras capture probe set | Gly12Arg | 1193 | |
| | Gly12Cys | 1887 | |
| | Gly12Ser | 3925 | O |
| | Wild type | 1261 | |

The detection results are shown in Table 9. In the cases where the EGFR capture probe set was used, the wild-type probe showed the strongest signal intensity. Therefore, it is thought that A549 cells do not have the T790M mutation. In the cases where the K-RAS capture probes were used, the Gly12Ser probe showed the strongest signal intensity. A549 cells are known to have the Gly12Ser mutation, and the present detection results were consistent with this.

Thus, by using the sandwich hybridization, the SNPs could be properly detected without performing PCR amplification.

### EXAMPLE 4

Although the K-ras gene mutations and EGFR gene mutation shown in Example 3 are important indices for administration of anticancer agents, it is also important to confirm the presence/absence of expression of these genes by another method. The present invention can directly detect RNAs, and can also detect mutations in the RNAs. Conventional RNA techniques require preparation of cDNA using reverse transcriptase before amplification by PCR. In general, since the reverse transcriptase used for reverse transcription easily causes mutations, it may cause false detection. The details are described below, but the present invention is not limited by the Example below.

### (Design of Capture Probes and Detection Probes)

The same capture probe sets and detection probes as in Example 3 were used.

### (Preparation of DNA Chip)

A DNA chip on which all capture probes in Table 6 are immobilized was prepared. The method of preparing the DNA chip was the same as in Example 1.

### (Preparation of Sample DNA)

For the sample DNA, total RNA extracted from A549 cells was used. A549 cells are cultured cells derived from a lung cancer tissue, and known to have the Gly12Ser mutation. By an ultrasonic treatment (Covaris Inc., s220), 5 µg of total RNA extracted from A549 cells was fragmented. The fragmentation treatment was carried out under the conditions for the method recommended by the manufacturer. The lengths of fragments were evaluated using a Bioanalyzer (Agilent). As a result, the mode of the nucleic acid length was 250 bases. The whole cleaved RNA was used as the sample RNA.

### (Preparation of Detection Probe Solution)

The detection probes were mixed together, and diluted with sterile water such that the concentration of each detection probe was 100 fmol/µL.

### (Hybridization)

To 5 µL of the sample RNA, 1 µL of the detection probe dilution was added, and the resulting mixture was heated using a thermal cycler at 95°C for 5 minutes. Thereafter, the solution was left to stand on the bench for 2 minutes to allow the solution to cool to room temperature. To this solution, 35 µL of a hybridization solution (1 wt% BSA (bovine serum albumin), 5×SSC, 0.1 wt% SDS (sodium dodecyl sulfate), 0.01 wt% salmon sperm DNA solution) was added to provide a hybridization solution. The whole solution was injected into the DNA chip, and the DNA chip was placed in an incubator heated at 32°C. Hybridization was carried out according to the standard protocol for "3D-Gene" with rotary shaking at 250 rpm at 32°C for 2 hours. Thereafter, the DNA chip was washed for 5 minutes with a washing liquid (0.5×SSC, 0.1 wt% SDS (sodium dodecyl sulfate)) heated at 30°C, and dried using a spin dryer (Wakenyaku Co., Ltd.). A solution prepared by adding streptavidin-phycoerythrin (ProZyme, Inc.) to a staining solution (50 ng/µl streptavidin-phycoerythrin, 100 mM MES, 1 M NaCl, 0.05 wt% Tween 20, 2 mg/ml BSA (bovine serum albumin)) was provided, and the solution was dropped on the DNA chip. The DNA chip was then incubated at 35°C for 5 minutes. After washing the DNA chip for 5 minutes with a washing liquid (6×SSPE, 0.01 wt% Tween 20) heated at 30°C, the DNA chip was dried using a spin dryer (Wakenyaku Co., Ltd.). The DNA chip after staining was subjected to detection of fluorescence signals using a DNA chip scanner (Toray Industries, Inc.). In terms of settings of the scanner, the laser output was set to 100%, and the photomultiplier voltage was set to 70%.

The detection results are shown in Table 10. In the cases where the EGFR capture probe set was used, the wild-type probe showed the strongest signal intensity. Therefore, it is thought that A549 cells do not have the T790M mutation. In the cases where the K-RAS capture probes were used, the Gly12Ser probe showed the strongest signal intensity. A549 cells are known to have the Gly12Ser mutation, and the present detection results were consistent with this.

**[Table 10]**

| | Mutation | Signal intensity | Probe with the strongest signal in the capture probe set |
|---|---|---|---|
| EGFR capture probe set | Control (g) | 857 | |
| | Control (a) | 793 | |
| | T790M | 756 | |
| | Wild type | 1193 | O |
| K-ras capture probe set | Gly 12Arg | 1115 | |
| | Gly12Cys | 1591 | |
| | Gly12Ser | 3060 | O |
| | Wild type | 1267 | |

Thus, by using the sandwich hybridization, the RNA could be directly detected and the SNPs could be properly detected without performing reverse transcription of the RNA.

### EXAMPLE 5

The present invention is described in more detail by way of another embodiment of detection of human papillomavirus. In the Example 1 and Example 2 described above, human papillomavirus DNA was detected. When nucleic acid is extracted from a human specimen and analyzed, the absence of a detection signal means either the absence of human papillomavirus in the human specimen, or failure of the operation of extraction or detection of the nucleic acid. It is difficult to distinguish between these. Such a problem can be solved by use of an internal standard.

It is known that, in cases where human papillomavirus DNA is extracted from a human specimen, the obtained sample solution contains a small amount of human DNA. From a human specimen uninfected with human papillomavirus, human papillomavirus DNA cannot be obtained, but human DNA can be obtained. By taking advantage of this fact, human DNA contained in the sample solution obtained by extraction from a human specimen can be used as an internal standard for detection of human papillomavirus DNA. In the present Example, a plasmid DNA containing a base sequence of papillomavirus incorporated therein, and human genomic DNA are mixed together to provide a sample for simulation. However, the present invention is not limited by the Example below.

### (Design of Capture Probes and Detection Probes)

Similarly to Example 1, sequences reported in the literature (J. Clin. Microbiol., 1995. pp. 901-905, Table 1) were employed for capture probes. In general, the binding strength in nucleic acid hybridization increases as the length of the nucleic acid increases. In view of this, as detection probes, sequences each having a length of 80 to 86 bases were provided for 5 regions located upstream and downstream of the capture probe as shown in Fig. 4 and Table 4.

As the capture probes having the sequences described above, synthetic DNAs modified with an amino group at the 5'-end were synthesized by Operon Biotechnologies, Inc. As the detection probes, those internally labeled with biotin were synthesized by Operon Biotechnologies, Inc. (Table 4).

Table 11 shows a capture probe and detection probes for Alu sequences, which are repetitively present in the human genome. As the capture probe having the sequence described above, a synthetic DNA modified with an amino group at the 5'-end was synthesized by Operon Biotechnologies, Inc. As the detection probes, those labeled with biotin at the 5'-end and 3'-end were synthesized by Operon Biotechnologies, Inc.

### (Preparation of DNA Chip)

A DNA chip on which all capture probes in Table 1 and the capture probe having the sequence of SEQ ID NO:46 shown in Table 11 are immobilized was prepared. The method of preparing the DNA chip was the same as in Example 1.

**[Table 11]**

| | Probe name | SEQ ID NO | Sequence 5'→3' |
|---|---|---|---|
| Detection probe | Alu detection probe 1 | SEQ ID NO:43 | GCTCACGCCTGTAATCCCAGCACTTTGGGA |
| | Alu detection probe 2 | SEQ ID NO:44 | CGGTGAAACCCCGTCTCTACTAAAAATACA |
| | Alu detection probe 3 | SEQ ID NO:45 | ACTCGGGAGGCTGAGGCAGGAGAATGGCG |
| Capture probe | Probe Alu | SEQ ID NO:46 | CGGATCACGAGGTCAGGAGATCGAGACCATCCTGGCTAAC |

### (Preparation of Sample DNA)

For the sample DNA, a recombinant plasmid pHPV16, which contains a cloned genomic DNA of human papillomavirus, was purchased from Health Science Research Resources Bank (Japan Health Sciences Foundation) and used. The total length of pHPV16 was 16,600 base pairs. If the molecular weight of one base pair is regarded as 680, 1 µg corresponds to 89 nmol. Human genomic DNA was purchased from Clontech.

The method for preparing the sample DNA was as follows. By an ultrasonic treatment (Covaris Inc., s220), 5 µg of pHPV16 and the human genomic DNA were fragmented. The fragmentation treatment was carried out under conditions where fragments having a length of 250 bases can be obtained according to the method recommended by the manufacturer. The lengths of fragments were evaluated using a Bioanalyzer (Agilent). As a result, the peak top value of the fragmented nucleic acid obtained under the treatment conditions was 250 bases. The concentration of the cleaved sample DNA was measured using NanoDrop (Thermo Fisher Scientific K.K., ND-1000) to determine the nucleic acid concentration. In consideration of the nucleic acid concentration, the cleaved DNA contained in each solution after cleavage was diluted such that pHPV16 was contained at 1 amol/µL and human genomic DNA was contained at 5 ng/µL using 1 ×hybridization solution.

### (Preparation of Detection Probe Solution)

The detection probes shown in Table 4 and Table 11 were dissolved in sterile water, and mixed together such that the concentration of each probe was 100 fmol/µL, to provide a detection probe liquid.

### (Hybridization)

To 5 µL of the sample DNA prepared by mixing the cleaved pHPV16 and human genomic DNA according to the composition shown in Table 12, 1 µL of the diluted detection probe liquid was added, and the resulting mixture was heated using a thermal cycler at 95°C for 5 minutes. Thereafter, the solution was left to stand on the bench for 2 minutes to allow the solution to cool to room temperature. To this solution, 35 µL of 1 ×hybridization solution (1 wt% BSA (bovine serum albumin), 5×SSC, 1 wt% SDS (sodium dodecyl sulfate), 50 ng/ml salmon sperm DNA solution, 5 wt% dextran sulfate sodium, 30% formamide) was added to provide a hybridization solution. The whole solution was injected into the DNA chip, and the DNA chip was placed in an incubator heated at 32°C. Hybridization was carried out according to the standard protocol for "3D-Gene" with rotary shaking at 250 rpm at 32°C for 2 hours. Thereafter, the DNA chip was washed for 5 minutes with a washing liquid (0.5×SSC, 0.1 wt% SDS (sodium dodecyl sulfate)) heated at 30°C, and dried using a spin dryer (Wakenyaku Co., Ltd.). A solution prepared by adding streptavidin-phycoerythrin (ProZyme, Inc.) to a staining solution (50 ng/µl streptavidin-phycoerythrin, 100 mM MES, 1 M NaCl, 0.05 wt% Tween 20, 2 mg/ml BSA (bovine serum albumin)) was provided, and the solution was dropped on the DNA chip. The DNA chip was then incubated at 35°C for 5 minutes. After washing the DNA chip for 5 minutes with a washing liquid (6×SSPE, 0.01 wt% Tween 20) heated at 30°C, the DNA chip was dried using a spin dryer (Wakenyaku Co., Ltd.). The DNA chip after staining was subjected to detection of fluorescence signals using a DNA chip scanner (Toray Industries, Inc.). In terms of settings of the scanner, the laser output was set to 100%, and the photomultiplier voltage was set to 70%.

The detection results are shown in Table 12.

**[Table 12]**

| Values of signals detected from Samples 1, 2 and 3 | | | |
|---|---|---|---|
| | Sample 1 (5 ng of human genomic DNA) | Sample 2 (5 ng of human genomic DNA + 1 amol of pHPV16) | Sample 3 (1 amol of pHPV16) |
| Capture probe Probe 16 | 0 | 15,000 | 15,000 |
| Capture probe Probe Alu | 5,000 | 5,000 | 0 |

In Sample 1 (human genomic DNA, 5 ng), a signal was detected only with the capture probe Probe Alu. Since this sample did not contain pHPV16 DNA, no signal was obtained with the capture probe Probe 16.

In Sample 3 (pHPV16, 1 amol), a signal was detected only with the capture probe Probe 16. Since this sample did not contain human genomic DNA, no signal was obtained with the capture probe Probe Alu. From these results, it can be seen that the capture probe and detection probe for pHPV16, and the detection probe and capture probe for Alu sequences do not cause erroneous hybridization with each other.

In Sample 2 (pHPV16, 1 amol; human genomic DNA, 5 ng), signals were detected with the capture probes Probe 16 and Probe Alu. Thus, it was found that pHPV16 and human genomic DNA can be detected simultaneously.

Thus, irrespective of whether human papillomavirus DNA is present or absent, Alu sequences in human DNA contained in the sample solution could be detected, and it was therefore found that human DNA can be used as an internal standard. It is thought that the present technique is effective also in cases where nucleic acid is extracted from a human specimen and analyzed.

## Claims

1. A method for detecting a target nucleic acid, said method comprising the steps of:
sequentially or simultaneously bringing a fragmentation product of a target nucleic acid, a plurality of detection probes, and a capture probe immobilized on a support, into contact with each other to hybridize said capture probe with said fragmentation product of said target nucleic acid and to hybridize said fragmentation product of said target nucleic acid with said plurality of detection probes, thereby binding said plurality of detection probes to said support through said capture probe and said fragmentation product of said target nucleic acid; and
detecting said plurality of detection probes bound to said support,
wherein in said fragmentation product of said target nucleic acid to be hybridized with said capture probe, a plurality of detection probes are hybridized at distances of not more than the mode of the nucleic acid length of said fragmentation product of said target nucleic acid from the binding position of said capture probe, the mode of the nucleic acid length of said fragmentation product of said target nucleic acid to be hybridized with said capture probe being within the range of 100 bases to 1500 bases, and
wherein the mode of the nucleic acid length is the x-axis value of the highest position in the waveform (peak top) of an electropherogram obtained using an electrophoretic method.

2. The method according to claim 1, wherein the step of sequentially or simultaneously bringing a fragmentation product of the target nucleic acid, a plurality of detection probes, and a capture probe immobilized on a support, into contact with each other is sequentially carried out by hybridizing said fragmentation product of said target nucleic acid with said plurality of detection probes and then hybridizing said fragmentation product of said target nucleic acid hybridized with said plurality of detection probes with said capture probe.

3. The method according to claims 1 or 2, wherein, in said fragmentation product of said target nucleic acid to be hybridized with said capture probe, a plurality of detection probes are hybridized at distances of not more than 1500 bases from the binding position of said capture probe.

4. The method according to any one of claims 1 to 3, wherein a fragmentation product of said target nucleic acid, prepared by carrying out a fragmentation treatment of said target nucleic acid such that the mode of the nucleic acid length is within the range of 100 bases to 1500 bases, is hybridized with said capture probe.

5. The method according to any one of claims 1 to 4, wherein said fragmentation product of said target nucleic acid to be hybridized with said capture probe has not undergone amplification by a nucleic acid amplification method.

6. The method according to any one of claims 1 to 5, wherein an animal-derived sample containing said target nucleic acid is subjected to said detection method, said detection method further comprising a step of detecting at least one type of repetitive sequences present in the animal genome as an internal standard, said repetitive sequences being contained in fragments of said animal genome.

7. The method according to claim 6, further comprising a step of fragmenting said animal genome, wherein said repetitive sequences are contained in the fragmented animal genome.

8. The method according to claim 6 or 7, wherein said animal is human.

9. The method according to any one of claims 6 to 8, wherein said repetitive sequences are short interspersed nuclear elements.

10. The method according to claim 9, wherein said short interspersed nuclear elements are Alu sequences.

## Patentansprüche

1. Verfahren zum Detektieren einer Zielnukleinsäure, wobei das Verfahren die folgenden Schritte umfasst:
sequentielles oder gleichzeitiges In-Kontakt-Bringen miteinander von einem Fragmentierungsprodukt einer Zielnukleinsäure, einer Vielzahl von Detektionssonden und einer auf einem Träger immobilisierten Fangsonde, um die Fangsonde mit dem Fragmentierungsprodukt der Zielnukleinsäure zu hybridisieren und das Fragmentierungsprodukt der Zielnukleinsäure mit der Vielzahl von Detektionssonden zu hybridisieren, wodurch die Vielzahl von Detektionssonden durch die Fangsonde und das Fragmentierungsprodukt der Zielnukleinsäure an den Träger gebunden wird; und
Detektieren von der Vielzahl von Detektionssonden, die an den Träger gebunden sind,
wobei in dem Fragmentierungsprodukt der mit der Fangsode zu hybridisierenden Zielnukleinsäure eine Vielzahl von Detektionssonden in Abständen von nicht mehr als dem Modus der Nukleinsäurelänge des Fragmentierungsprodukts der Zielnukleinsäure zu der Bindungsposition der Fangsonde hybridisiert wird, wobei der Modus der Nukleinsäurelänge des Fragmentierungsprodukts der mit der Fangsonde zu hybridisierenden Zielnukleinsäure in dem Bereich von 100 Basen bis 1500 Basen liegt, und
wobei der Modus der Nukleinsäurelänge der x-Achsenwert der höchsten Position in der Wellenform (Peakspitze) eines Elektropherogramms ist, das unter Verwendung eines elektrophoretischen Verfahrens erhalten wurde.

2. Verfahren nach Anspruch 1, wobei der Schritt des sequentiellen oder gleichzeitigen In-Kontakt-Bringens miteinander von einem Fragmentierungsprodukt der Zielnukleinsäure, einer Vielzahl von Detektionssonden und einer auf einem Träger immobilisierten Fangsonde sequenziell durchgeführt wird, indem das Fragmentierungsprodukt der Zielnukleinsäure mit der Vielzahl von Detektionssonden hybridisiert und dann das Fragmentierungsprodukt der Zielnukleinsäure, das mit der Vielzahl von Detektionssonden hybridisiert ist, mit der Fangsonde hybridisiert wird.

3. Verfahren nach Anspruch 1 oder 2, wobei in dem Fragmentierungsprodukt der mit der Fangsonde zu hybridisierenden Zielnukleinsäure eine Vielzahl von Detektionssonden in Abständen von nicht mehr als 1500 Basen zu der Bindungsposition der Fangsonde hybridisiert wird.

4. Verfahren nach einem der Ansprüche 1 bis 3, wobei ein Fragmentierungsprodukt der Zielnukleinsäure, die durch das Durchführen einer Fragmentierungsbehandlung der Zielnukleinsäure hergestellt wird, so dass der Modus der Nukleinsäurelänge innerhalb des Bereichs von 100 Basen bis 1500 Basen liegt, mit der Fangsonde hybridisiert wird.

5. Verfahren nach einem der Ansprüche 1 bis 4, wobei das Fragmentierungsprodukt der mit der Fangsonde zu hybridisierenden Zielnukleinsäure keine Amplifikation mittels eines Nukleinsäureamplifikationsverfahrens durchlaufen hat.

6. Verfahren nach einem der Ansprüche 1 bis 5, wobei eine aus einem Tier stammende Probe, die die Zielnukleinsäure enthält, dem Detektionsverfahren unterzogen wird, wobei das Detektionsverfahren ferner einen Schritt des Detektierens von mindestens einem Typ repetitiver Sequenzen, die im Tiergenom als interner Standard vorhanden sind, umfasst, wobei die repetitiven Sequenzen in Fragmenten des Tiergenoms enthalten sind.

7. Verfahren nach Anspruch 6, ferner umfassend einen Schritt des Fragmentierens des tierischen Genoms, wobei die repetitiven Sequenzen in dem fragmentierten tierischen Genom enthalten sind.

8. Verfahren nach Anspruch 6 oder 7, wobei das Tier ein Mensch ist.

9. Verfahren nach einem der Ansprüche 6 bis 8, wobei die repetitiven Sequenzen kurze, eingestreute Kernsequenzelemente (Englisch: short interspersed nuclear elements) sind.

10. Verfahren nach Anspruch 9, wobei die kurzen, eingestreuten Kernsequenzelemente Alu-Sequenzen sind.

## Revendications

1. Procédé pour détecter un acide nucléique cible, ledit procédé comprenant les étapes consistant à :
mettre en contact les uns avec les autres, séquentiellement ou simultanément, un produit de fragmentation d'un acide nucléique cible, une pluralité de sondes de détection, et une sonde de capture immobilisée sur un support, afin d'hybrider ladite sonde de capture avec ledit produit de fragmentation dudit acide nucléique cible et d'hybrider ledit produit de fragmentation dudit acide nucléique cible avec ladite pluralité de sondes de détection, en liant ainsi ladite pluralité de sondes de détection audit support par l'intermédiaire de ladite sonde de capture et dudit produit de fragmentation dudit acide nucléique cible ; et
détecter ladite pluralité de sondes de détection liées audit support,
dans lequel, dans ledit produit de fragmentation dudit acide nucléique cible devant être hybridé avec ladite sonde de capture, une pluralité de sondes de détection sont hybridées à des distances n'excédant pas le mode de la longueur d'acide nucléique dudit produit de fragmentation dudit acide nucléique cible à partir de la position de liaison de ladite sonde de capture, le mode de la longueur d'acide nucléique dudit produit de fragmentation dudit acide nucléique cible devant être hybridé avec ladite sonde de capture étant compris dans la plage de 100 bases à 1 500 bases, et
dans lequel le mode de la longueur d'acide nucléique est la valeur de l'axe x de la position la plus élevée dans la courbe (sommet du pic) d'un électrophérogramme obtenu en utilisant un procédé électrophorétique.

2. Procédé selon la revendication 1, dans lequel l'étape consistant à mettre en contact les uns avec les autres, séquentiellement ou simultanément, un produit de fragmentation de l'acide nucléique cible, une pluralité de sondes de détection, et une sonde de capture immobilisée sur un support, est réalisée séquentiellement en hybridant ledit produit de fragmentation dudit acide nucléique cible avec ladite pluralité de sondes de détection puis en hybridant ledit produit de fragmentation dudit acide nucléique cible hybridé avec ladite pluralité de sondes de détection avec ladite sonde de capture.

3. Procédé selon les revendications 1 ou 2, dans lequel, dans ledit produit de fragmentation dudit acide nucléique cible devant être hybridé avec ladite sonde de capture, une pluralité de sondes de détection sont hybridées à des distances n'excédant pas 1 500 bases à partir de la position de liaison de ladite sonde de capture.

4. Procédé selon l'une quelconque des revendications 1 à 3, dans lequel un produit de fragmentation dudit acide nucléique cible, préparé en réalisant un traitement de fragmentation dudit acide nucléique cible de sorte que le mode de la longueur d'acide nucléique soit compris dans la plage de 100 bases à 1500 bases, est hybridé avec ladite sonde de capture.

5. Procédé selon l'une quelconque des revendications 1 à 4, dans lequel ledit produit de fragmentation dudit acide nucléique cible devant être hybridé avec ladite sonde de capture n'a pas subi d'amplification par un procédé d'amplification d'acide nucléique.

6. Procédé selon l'une quelconque des revendications 1 à 5, dans lequel un échantillon dérivé d'un animal contenant ledit acide nucléique cible est soumis audit procédé de détection, ledit procédé de détection comprenant en outre une étape consistant à détecter au moins un type de séquences répétées présentes dans le génome animal en tant qu'étalon interne, lesdites séquences répétées étant contenues dans des fragments dudit génome animal.

7. Procédé selon la revendication 6, comprenant en outre une étape consistant à fragmenter ledit génome animal, dans lequel lesdites séquences répétées sont contenues dans le génome animal fragmenté.

8. Procédé selon la revendication 6 ou 7, dans lequel ledit animal est humain.

9. Procédé selon l'une quelconque des revendications 6 à 8, dans lequel lesdites séquences répétées sont des petits éléments nucléaires intercalés.

10. Procédé selon la revendication 9, dans lequel lesdits petits éléments nucléaires intercalés sont des séquences Alu.
